# EUROPÄISCHE PATENTSCHRIFT

(11) **EP 3 223 782 B1**
(45) Veröffentlichungstag und Bekanntmachung des Hinweises auf die Patenterteilung: **04.12.2019**
(21) Anmeldenummer: 15780857.7
(22) Anmeldetag: 14.10.2015
(51) Int. Cl.: A61K 8/39, A61K 8/41, A61K 8/49, A61Q 5/06, C09B 31/14

(54) **MITTEL ZUM FÄRBEN VON KERATINISCHEN FASERN, ENTHALTEND MINDESTENS EINEN DIMEREN, DIKATIONISCHEN AZOFARBSTOFF UND MINDESTENS EIN ANIONISCHES UND/ODER KATIONISCHES TENSID**
AGENTS FOR DYEING KERATIN FIBRES, CONTAINING AT LEAST ONE DIMERIC, DICATIONIC AZO DYE AND AT LEAST ONE ANIONIC AND/OR CATIONIC SURFACTANT
AGENTS DE COLORATION DE FIBRES KÉRATINIQUES, CONTENANT AU MOINS UN COLORANT AZOÏQUE DICATIONIQUE DIMÈRE ET AU MOINS UN TENSIOACTIF ANIONIQUE ET/OU CATIONIQUE

(30) Priorität: 25.11.2014 DE 102014223935
(43) Veröffentlichungstag der Anmeldung: 04.10.2017
(73) Patentinhaber: Henkel AG & Co. KGaA, 40589 Düsseldorf (DE)
(72) Erfinder: GEBERT-SCHWARZWÄLDER, Antje, 41469 Neuss (DE); GIESA, Helmut, 40670 Meerbusch (DE); KROOS, Astrid, 40789 Monheim (DE)
(86) Internationale Anmeldenummer: PCT/EP2015/073775
(87) Internationale Veröffentlichungsnummer: WO 2016/083012

(56) Entgegenhaltungen:
- EP-A1- 0 531 943
- EP-A1- 0 994 691
- EP-A1- 1 448 156
- EP-A2- 1 416 908
- EP-A2- 1 483 334
- DE-A1- 4 128 490
- FR-A1- 2 915 681
- US-A- 4 607 071
- US-A1- 2004 200 009

## Beschreibung

Die vorliegende Erfindung betrifft Mittel zum Färben von keratinischen Fasern, insbesondere menschlichen Haaren, die (a) mindestens einen dimeren, dikationischen Azofarbstoff einer speziellen Formel (Ia) in Kombination mit (b) mindestens einem anionischen und/oder kationischen Tensid enthalten.

Es hat sich herausgestellt, dass durch den Einsatz von speziellen anionischen Tensiden (b) die Farbintensität von kationischen Azofarbstoffen der Formel (Ia) erhöht werden kann. Ein weiterer Gegenstand der vorliegenden Erfindung ist daher die Verwendung von anionischen Tensiden (b) zur Verbesserung des Farbaufzugsvermögens von kationischen Azofarbstoffen der Formel (Ia), auf keratinischen Fasern. Weiterhin hat sich herausgestellt, dass auch durch den Einsatz von speziellen kationischen Tensiden (b) die Farbintensität von kationischen Azofarbstoffen der Formel (Ia), erhöht werden kann. Ein weiterer Gegenstand der vorliegenden Erfindung ist daher die Verwendung von speziellen kationischen Tensiden (b) zur Verbesserung des Farbaufzugsvermögens von kationischen Azofarbstoffen der Formel (Ia) auf keratinischen Fasern.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen.

Abhängig vom gewünschten Farbergebnis setzt der Fachmann direktziehende Farbstoffe unterschiedlicher Farbstoffklassen ein. Die aus dem Stand der Technik bekannten direktziehenden Farbstoffe gehören beispielsweise zur Klasse der Nitrofarbstoffe, der Anthrachinonfarbstoffe, der Azofarbstoffe, der Triarylmethanfarbstoffe oder der Methinfarbstoffe. Alle diese Farbstoffklassen sollten für die Anwendung im Kosmetikbereich ein bestimmtes Anforderungsprofil erfüllen. So sollten direktziehende Farbstoffe ein intensives Färbeergebnis liefern und möglichst gute Echtheitseigenschaften besitzen. Durch Umwelteinflüsse sollte das mit direktziehenden Farbstoffen erhaltene Farbergebnis möglichst wenig beeinflusst werden, d.h. die Farbstoffe sollten beispielsweise eine gute Waschechtheit, Lichtechtheit und Reibechtheit besitzen. Auch chemische Einflüsse, welchen die keratinischen Fasern nach dem Färbeprozess ausgesetzt sein können (wie beispielsweise Dauerwellen), sollten das Farbergebnis möglichst wenig verändern.

Um gleichzeitig mit der Färbung auch eine Aufhellung zu erreichen, sollten die direktziehenden Farbstoffe nach Möglichkeit auch mit den bei Blondierverfahren üblicherweise eingesetzten Oxidationsmitteln (wie z.B. Wasserstoffperoxid und/oder Persulfate) kompatibel sein.

Für die starke Aufhellung von dunklen Haaren wird nicht nur Wasserstoffperoxid allein, sondern eine Kombination aus Wasserstoffperoxid und Persulfaten (z.B. Ammoniumpersulfat, Kaliumpersulfat und/oder Natriumpersulfat) eingesetzt. Soll also dunkles Haar in einem Schritt stark aufgehellt und gleichzeitig in einem leuchtenden Farbton gefärbt werden, ist der Einsatz einer Mischung aus Wasserstoffperoxid, Persulfaten und einem direktziehenden Farbstoff von Vorteil. Obwohl dem Fachmann zum Färben von Haaren viele intensiv färbende direktziehende Farbstoffe bekannt sind, so kennt er doch nur eine sehr begrenzte Auswahl an Farbstoffen, die die starken oxidativen Bedingungen, wie sie eine Mischung der oben genannten Oxidationsmittel darstellt, ohne Zersetzung überstehen. Zudem weisen die aus dem Stand der Technik bekannten oxidationsstabilen Farbstoffe im Hinblick auf ihre übrigen Echtheitseigenschaften gravierende Nachteile auf.

Für das gleichzeitige Färben und starke Aufhellen von Haaren besteht damit nach wie vor ein Bedarf an Farbstoffen mit hoher Stabilität gegenüber starken Oxidationsmitteln. Auch unter diesen extremen Anwendungsbedingungen sollen diese Farbstoffe ihre positiven Echtheits- und Färbeeigenschaften nicht verlieren.

Es hat sich gezeigt, dass leuchtende und intensive Färbungen insbesondere mit kationischen direktziehenden Farbstoffen erzeugt werden können. Kationische Farbstoffe zeichnen sich oft durch eine besonders hohe Affinität zu Keratinfasern aus, was auf die Wechselwirkungen der positiven Ladungen der Farbstoffe mit negativ geladenen Strukturbestandteilen der keratinischen Fasern zurückgeführt werden kann. Daher lassen sich mit kationischen Farbstoffen oftmals besonders intensive Färbungen erzielen.

Aus dem Stand der Technik hinlänglich bekannte monomere kationische Azofarbstoffe sind beispielsweise die Vertreter Basic Orange 31 (Alternativname: 2-[(4-Aminophenyl)azo]-1,3-dimethyl-1H-imidazolium chloride, CAS-Nr. 97404-02-9) und Basic Red 51 (Alternativname: 2-[((4-Dimethylamino)phenyl)azo]-1,3-dimethyl-1H-imidazolium chlorid, CAS-Nr. 77061-58-6).

Beide Farbstoffe färben keratinischen Fasern mit herausragender Farbintensität in orange bis roten Nuancenbereich. Es besteht weiterhin auch noch Bedarf an direktziehenden Blaufarbstoffen, welche mit diesen beiden Farbstoffen in optimaler Weise kompatibel sind.

US 2004/200009 A1 offenbart verschiedene dimere Azofarbstoffe mit 1,2,4-Triazol-Endgruppen.

WO 03/076518 A2 beschreibt Azofarbstoffe, die auch zur Färbung von Keratinfasern eingesetzt werden können.

Gegenstand der WO 03/032937 A1 ist ein Verfahren zum Färben von Haaren, bei dem ein Farbstoff der Formel (1) eingesetzt wird. Bei dem Farbstoff der Formel (1) handelt es sich um einen dimeren Azofarbstoff, der Imidazol-Endgruppen trägt.

WO 02/100366 A1 beansprucht Mittel zum Färben von Haaren, die u.a. dimere kationische Azofarbstoffe der Formel (I) oder (II) enthalten. Hierbei umfassen die Farbstoffe der Formeln (I) und (II) eine Imidazolendgruppe.

FR 2 915 681 A1 betrifft ein Verfahren zum Färben von Keratinfasern, bei dem polykationische Farbstoffe mit mindestens 2 positiven Ladungen eingesetzt werden.

Aufgabe der vorliegenden Anmeldung ist es daher, Färbemittel für keratinische Fasern, insbesondere menschliche Haare, bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Im Falle der gleichzeitigen Anwendung mit Oxidationsfarbstoffen und/oder Oxidationsmitteln sollen die direktziehenden Farbstoffe eine hohe Stabilität gegen Wasserstoffperoxid und andere Oxidationsmittel aufweisen und ihre positiven Echtheits- und Färbeeigenschaften nicht verlieren. Zudem sollten möglichst leuchtende und intensive Färbungen im Rotbereich erzielt werden.

Darüber hinaus sollen die vorgenannten Farbstoffe auch besonders gut mit den kationischen Azofarbstoffen Basic Orange 31 und Basic Red 51 kompatibel sein.

Überraschenderweise konnte nun gefunden werden, dass diese Aufgabe in hervorragendem Maße erfüllt wird, wenn Farbstoffe der nachfolgend beschriebenen Formel (Ia) in Kombination mit mindestens einem anionischen Tensid und/oder kationischen Tensid in Mitteln zum Färben von keratinischen Fasern eingesetzt werden.

Ein erster Gegenstand der vorliegenden Erfindung ist ein Mittel zum Färben von keratinischen Fasern, insbesondere menschlichen Haaren, enthaltend in einem kosmetischen Träger
(a) mindestens einen direktziehenden Farbstoff der Formel (Ia), 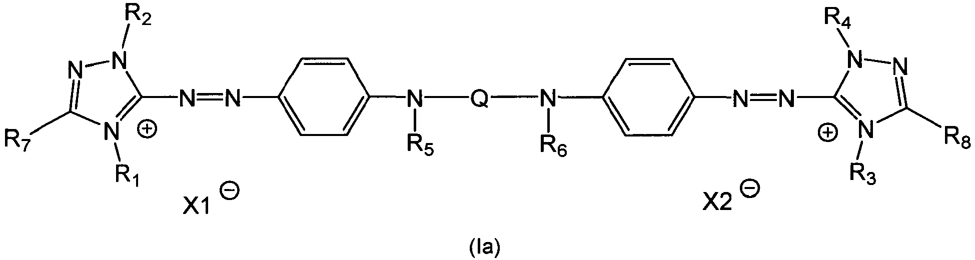 wobei
   - R1, R2, R3, R4: unabhängig voneinander für eine C₁-C₆-Alkylgruppe, eine C₂-C₆-Alkenylgruppe oder für eine Hydroxy-C₂-C₆-alkylgruppe stehen,
   - R5, R6: unabhängig voneinander für ein Wasserstoffatom, eine C₁-C₆-Alkylgruppe, eine C₂-C₆-Alkenylgruppe, eine Hydroxy-C₂-C₆-alkylgruppe oder für eine Cyano-C₁-C₆-alkylgruppe stehen,
   - R7, R8: unabhängig voneinander für ein Wasserstoffatom, eine C₁-C₆-Alkylgruppe, ein Halogenatom aus der Gruppe Chlor, Brom, Fluor oder Jod, oder für eine C₁-C₆-Alkoxygruppe stehen,
   - X1, X2: unabhängig voneinander für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, stehen,
   - Q: für eine Gruppierung der Formel (II) steht,

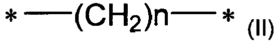

   - n: für eine ganze Zahl von 3 bis 6 steht,
   und
(b) mindestens ein anionisches Tensid und/oder mindestens ein kationisches Tensid.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, Mattierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen. Unter dem Begriff "Mattierung von Keratinfasern" wird das Entgegenwirken von unerwünschten Nuancenverschiebungen verstanden, welche bei oxidativen Farbveränderungen von Keratinfasern, insbesondere bei Blondierungen oder bei Bleichprozessen, auftreten. Ziel ist der Mattierung ist die Abschwächung des durch unvollständige Blondierung hervorgerufenen orangen bis rötlichen Farbeindrucks und das Erzeugen einer silbrig-kühlen Farbwahrnehmung nach dem Blondierprozess. Die bei der Mattierung eingesetzten Wirkstoffe können in Form eines Nachbehandlungsschrittes nach dem Blondieren bzw. Bleichen der Keratinfasern appliziert werden. Es ist aber ebenso möglich, die für die Mattierung eingesetzten Wirkstoffe im Rahmen eines einstufigen Verfahrens zusammen mit dem Blondiermittel bzw. dem Bleichmittel auf die Keratinfasern aufzutragen. Als für die Mattierung geeignete Wirkstoffe können direktziehende Farbstoffe - entweder allein oder im Farbstoffgemisch - mit den geeigneten farblichen Eigenschaften verwendet werden. Darüber hinaus ist es ebenfalls möglich, für die Mattierung direktziehende Farbstoffe in Kombination mit Oxidationsfarbstoffvorprodukten (Entwicklern und Kupplern) einzusetzen.

Die erfindungsgemäßen Mittel enthalten die direktziehenden Farbstoffe der Formel (Ia) in einem kosmetischen Träger. Dieser kosmetische Träger ist bevorzugt wässrig, alkoholisch oder wässrigalkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als ersten wesentlichen Inhaltsstoff (a) enthalten die erfindungsgemäßen Mittel mindestens einen Farbstoff der Formel (Ia).

Die Substituenten R1 bis R8 der Verbindungen der Formel (Ia) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Bevorzugte Beispiele für Cyano-C₁-C₆-alkylgruppen sind die Cyanomethylgruppe, die 2-Cyanoethylgruppe und die 3-Cyanopropylgruppe. Erfindungsgemäß bevorzugte Halogen-C₁-C₆-alkylgruppen sind die Chlormethylgruppe, die Brommethylgruppe, die Fluormethylgruppe, die 2-Chlorethylgruppe, die 2-Bromethylgruppe, die 2-Fluormethylgruppe, die 2-Chlorpropylgruppe, die 2-Brompropylgruppe, die 2-Fluropropylgruppe, die 3-Chlorpropylgruppe, die 3-Brompropylgruppe und die 3-Fluorpropylgruppe. Halogenatome sind ausgewählt aus der Gruppe Chlor, Brom, Fluor und/oder Jod, hierbei sind Chlor und Brom besonders bevorzugt. Beispielhaft für eine C1-C6-Alkoxygruppe können die Methoxy-, die Ethoxy- und die Propoxygruppe genannt werden.

Die Verbindungen der allgemeinen Formel (la) tragen die Reste R1, R2, R3 und R4; hierbei können die Reste R1 bis R4 gleich oder verschieden sein. Bevorzugt stehen R1 bis R4 unabhängig voneinander für eine für eine C₁-C₆-Alkylgruppe oder für eine C₂-C₆-Alkenylgruppe. Besonders bevorzugt stehen die Reste R1 bis R4 unabhängig voneinander für eine C₁-C₆-Alkylgruppe, insbesondere für eine Methylgruppe oder für eine Ethylgruppe.

In einer besonders bevorzugten Ausführungsform ist ein erfindungsgemäßes Mittel dadurch gekennzeichnet, dass es (a) mindestens einen direktziehenden Farbstoff der allgemeinen Formel (Ia) enthält, in der
- R1, R2, R3, R4: unabhängig voneinander für eine Methylgruppe oder für eine Ethylgruppe stehen.

Weiterhin tragen die Verbindungen der allgemeinen Formel (Ia) die Reste R5 und R6; hierbei können R5 und R6 gleich oder verschieden sein. Bevorzugt stehen R5, und R6 unabhängig voneinander für ein Wasserstoffatom, eine C₁-C₆-Alkylgruppe oder eine C₂-C₆-Alkenylgruppe.

Besonders bevorzugt stehen R5, und R6 unabhängig voneinander für ein Wasserstoffatom oder eine C₁-C₆-Alkylgruppe. Ganz besonders bevorzugt stehen R5 und R6 unabhängig voneinander für ein Wasserstoffatom, eine Methylgruppe oder eine Ethylgruppe.

In einer besonders bevorzugten Ausführungsform ist ein erfindungsgemäßes Mittel dadurch gekennzeichnet, dass es (a) mindestens einen direktziehenden Farbstoff der allgemeinen Formel (Ia) enthält, in der
- R5, R6: unabhängig voneinander für ein Wasserstoffatom, eine Methylgruppe oder für eine Ethylgruppe stehen.

In einer ganz besonders bevorzugten Ausführungsform ist ein erfindungsgemäßes Mittel dadurch gekennzeichnet, dass es (a) mindestens einen direktziehenden Farbstoff der allgemeinen Formel (Ia) enthält, in der
- R1, R2, R3, R4: unabhängig voneinander für eine Methylgruppe oder für eine Ethylgruppe stehen und
- R5, R6: unabhängig voneinander für ein Wasserstoffatom, eine Methylgruppe oder für eine Ethylgruppe stehen.

Weiterhin tragen die direktziehenden Farbstoffe der allgemeinen Formel (Ia) die Reste R7 und R8; auch die Reste R7 und R8 können gleich oder verschieden sein. Bevorzugt stehen R7, und R8 unabhängig voneinander für ein Wasserstoffatom, eine C₁-C₆-Alkylgruppe oder eine C₁-C₆-Alkoxygruppe. Besonders bevorzugt stehen R7 und R8 unabhängig voneinander für ein Wasserstoffatom, für eine Methylgruppe oder für eine Methoxygruppe. Ganz besonders bevorzugt stehen R7 und R8 beide für ein Wasserstoffatom.

In einer weiteren besonders bevorzugten Ausführungsform ist ein erfindungsgemäßes Mittel dadurch gekennzeichnet, dass es (a) mindestens einen direktziehenden Farbstoff der allgemeinen Formel (Ia) enthält, in der
- R7, R8: unabhängig voneinander für ein Wasserstoffatom, eine Methylgruppe oder für eine Methoxygruppe, bevorzugt für ein Wasserstoffatom, stehen.

Bei den erfindungsgemäßen Farbstoffen der Formel (Ia) handelt es sich um dimere Azofarbstoffe, die zweifach positiv geladen sind. Die beiden positiven Ladungen werden durch die anionschen Gegenionen X1 und X2 neutralisiert. Hierbei ist der dikationische organische Teil für die Blaufärbung der Keratinfasern verantwortlich. Die Gegenionen X1 und X2 dienen lediglich der Wahrung der Elektroneutralität, so dass die genaue Natur der Gegenionen X1 und X2 für die Erzielung des gewünschten Farbergebnisses keine wesentliche Rolle spielt. Da der Farbstoff in einem kosmetischen Mittel eingesetzt wird, müssen die Gegenionen X1 und X2 physiologisch verträglich sein. Physiologisch verträglich bedeutet in diesem Zusammenhang zum Einsatz im kosmetischen Mittel (d.h. zur Anwendung auf dem menschlichen Haar und der menschlichen Haut) geeignet. Bei X1 und X2 handelt es sich um physiologisch verträgliche Anionen, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat.

Unter Chlorid wird ein Anion Cl⁻ verstanden. Unter Bromid wird ein Anion Br verstanden. Unter lodid wird ein Anion J⁻ verstanden. Unter Methylsulfat wird ein Anion H₃COSO₄⁻ verstanden. Unter p-Toluolsulfonat wird ein Anion H₃C(C₆H₄)SO₃⁻ verstanden. Unter Acetat wird ein Anion H₃CCOO⁻ verstanden. Unter Hydrogensulfat wird ein Anion HSO₄⁻ verstanden.

Unter ½ Sulfat wird ein halbes Äquivalent des doppelt negativ geladenen Anions SO₄²⁻ verstanden. Unter ½ Tetrachlorozinkat wird ein halbes Äquivalent des doppelt negativ geladenen Anions ZnCl₄²⁻ verstanden. Bei Sulfat und Tetrachlorozinkat ist es demzufolge ebenfalls möglich und auch erfindungsgemäß, wenn der dikationische Farbstoff der Formel (Ia) durch ein SO₄²⁻ Ion bzw. durch ein ZnCl₄²⁻ Ion neutralisiert wird.

Bei der Gruppierung Q handelt es sich um eine Gruppierung, welche die beiden einfach positiv geladenen Chromophore des Farbstoffs zum Dimer verknüpft. Q steht für eine Gruppierung der Formel (II), 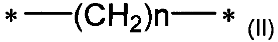
- n: für eine ganze Zahl von 3 bis 6 steht.

Die beiden mit einem Stern markierten Positionen stellen hierbei jeweils die Verknüpfungspositionen zu den beiden N-Atomen der Formel (Ia) dar.

Überraschenderweise hat es sich für die Erzielung eines intensiven Farbergebnisses als grundsätzlich wichtig und erfindungswesentlich herausgestellt, dass die verbindende Gruppierung Q, welche die beiden Azo-Chromophore miteinander verknüpft, eine Kettenlänge von mindestens 3 Atomen aufweist. Aus diesem Grund steht n in der Formel (II) für eine ganze Zahl von mindestens 3. Die verbindende Gruppierung Q der Formel (II) umfasst demnach mindestens 3 C-Atome (d.h. hierbei handelt es sich um eine Gruppierung mit der Mindestlänge von -CH₂-CH₂-CH₂-).

Im Rahmen von Vergleichsversuchen hat sich herausgestellt, dass dimere Azofarbstoffe des prinzipiellen Typs der Formel (Ia), die jedoch eine nicht erfindungsgemäße Linker-Gruppe Q mit einer Länge von nur 2 C-Atomen besitzen, ein extrem schlechtes Farbaufzugsvermögen auf die Keratinfasern besitzen.

Während mit den erfindungsgemäßen Farbstoffen der Formel (Ia) intensive Färbungen mit tief orangerotem bzw. kirschrotem oder granatrotem Farbton erzielt werden konnten, führten Färbungen mit analogen dimeren Farbstoffen, die über eine kürzere Gruppierung Q mit einer Kettenlänge von nur 2 C-Atomen verknüpft sind, zu praktisch überhaupt keinem Farbaufzug auf den keratinischen Fasern. Ohne auf eine Theorie beschränkt zu sein, könnte möglicherweise eine mit der kurzen Linker-Kette Q verbundene starre Geometrie und hierdurch bedingt eine ungünstige räumliche Konformation des Farbstoffes die Diffusion der kurzkettigen dimeren Farbstoffe in die keratinische Faser hinein verschlechtern.

In einer explizit ganz besonders bevorzugten Ausführungsform ist ein erfindungsgemäßes Mittel dadurch gekennzeichnet, dass es (a) mindestens einen Farbstoff der allgemeinen Formel (Ia) enthält, in der
- Q: für eine Gruppierung der Formel (II) steht, 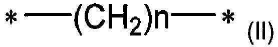
und
- n: für die Zahl 3 steht.

In einer weiteren bevorzugten Ausführungsform ist ein Mittel zum Färben von keratinischen Fasern dadurch gekennzeichnet, dass es mindestens eine Verbindung der allgemeinen Formel (Ia) enthält, die ausgewählt ist aus
- Salzen des 5-[2-(4-{[3-({4-[2-(1,4-Dimethyl-1H-1,2,4-triazol-4-ium-5-yl)diazen-1-yl]phenyl}amino)propyl]amino}phenyl)diazen-1-yl]-1,4-dimethyl-1H-1,2,4-triazol-4-iums 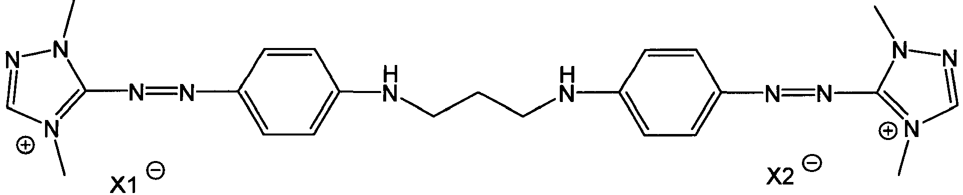
- Salzen des 5-[2-(4-{[4-({4-[2-(1,4-Dimethyl-1H-1,2,4-triazol-4-ium-5-yl)diazen-1-yl]phenyl}amino)butyl]amino}phenyl)diazen-1-yl]-1,4-dimethyl-1H-1,2,4-triazol-4-iums 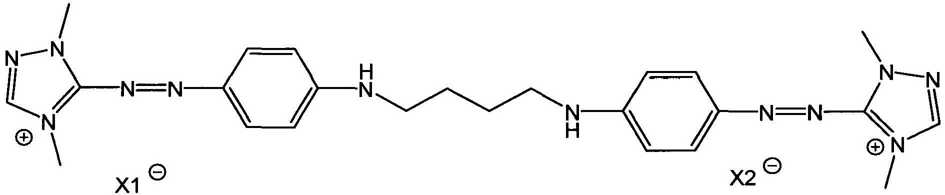
- Salzen des 5-[2-(4-{[5-({4-[2-(1,4-Dimethyl-1H-1,2,4-triazol-4-ium-5-yl)diazen-1-yl]phenyl}amino)pentyl]amino}phenyl)diazen-1-yl]-1,4-dimethyl-1H-1,2,4-triazol-4-iums 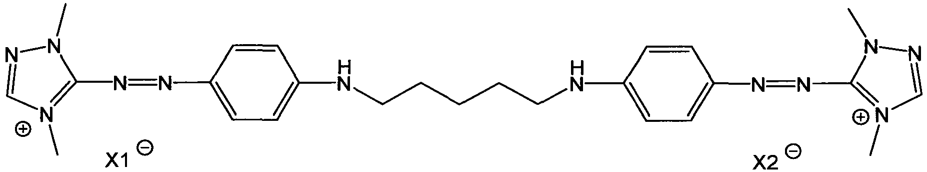
- Salzen des 5-[2-(4-{[3-({4-[2-(1,4-Dimethyl-1H-1,2,4-triazol-4-ium-5-yl)diazen-1-yl]phenyl}-(methyl)amino)propyl](methyl)amino}phenyl)diazen-1-yl]-1,4-dimethyl-1H-1,2,4-triazol-4-iums 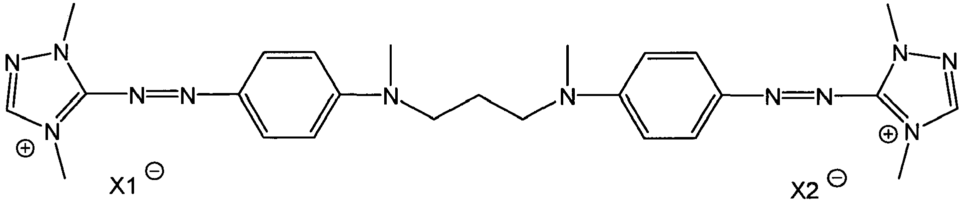
- Salzen des 5-[2-(4-{[4-({4-[2-(1,4-Dimethyl-1H-1,2,4-triazol-4-ium-5-yl)diazen-1-yl]phenyl}-(methyl)amino)butyl](methyl)amino}phenyl)diazen-1-yl]-1,4-dimethyl-1H-1,2,4-triazol-4-iums 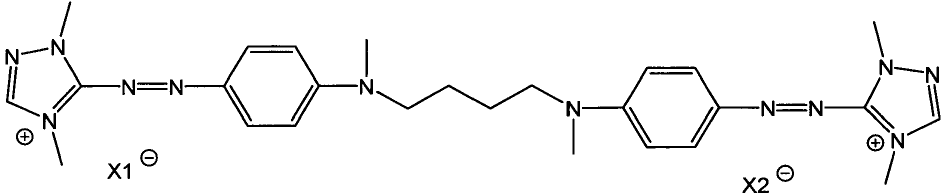
- Salzen des 5-[2-(4-{[5-({4-[2-(1,4-Dimethyl-1H-1,2,4-triazol-4-ium-5-yl)diazen-1-yl]phenyl}-(methyl)amino)pentyl](methyl)amino}phenyl)diazen-1-yl]-1,4-dimethyl-1H-1,2,4-triazol-4-iums 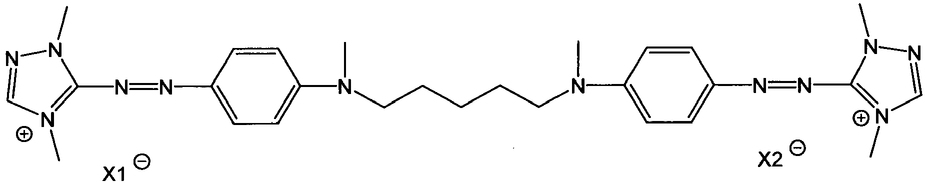
- Salzen des 5-[2-(4-{[3-({4-[2-(1,4-Dimethyl-1H-1,2,4-triazol-4-ium-5-yl)diazen-1-yl]phenyl}-(ethyl)amino)propyl](ethyl)amino}phenyl)diazen-1-yl]-1,4-dimethyl-1H-1,2,4-triazol-4-iums 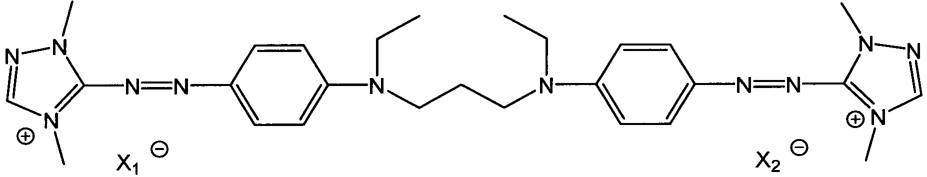
- Salzen des 5-[2-(4-{[4-({4-[2-(1,4-Dimethyl-1H-1,2,4-triazol-4-ium-5-yl)diazen-1-yl]phenyl}-(ethyl)amino)butyl](ethyl)amino}phenyl)diazen-1-yl]-1,4-dimethyl-1H-1,2,4-triazol-4-iums 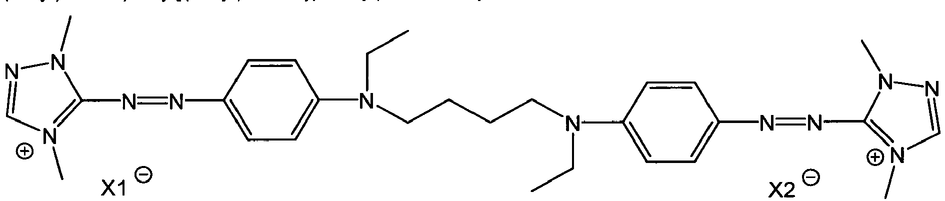
- Salzen des 5-[2-(4-{[5-({4-[2-(1,4-Dimethyl-1H-1,2,4-triazol-4-ium-5-yl)diazen-1-yl]phenyl}-(ethyl)amino)pentyl](ethyl)amino}phenyl)diazen-1-yl]-1,4-dimethyl-1H-1,2,4-triazol-4-iums 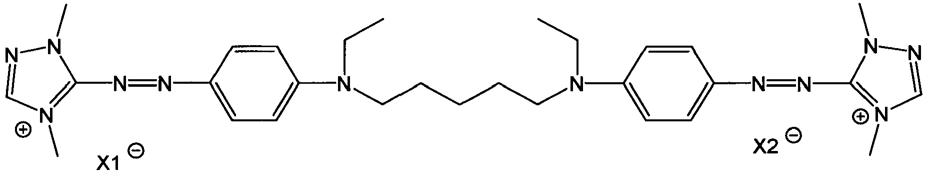
- Salzen des 5-[2-(4-{[3-({4-[2-(1,4-Diethyl-1H-1,2,4-triazol-4-ium-5-yl)diazen-1-yl]phenyl}-amino)propyl]amino}phenyl)diazen-1-yl]-1,4-diethyl-1H-1,2,4-triazol-4-iums 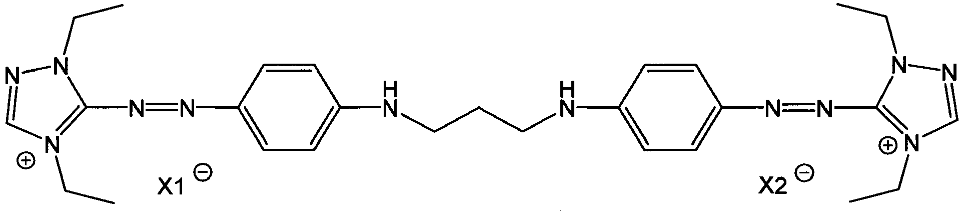
- Salzen des 5-[2-(4-{[4-({4-[2-(1,4-Diethyl-1H-1,2,4-triazol-4-ium-5-yl)diazen-1-yl]phenyl}-amino)butyl]amino}phenyl)diazen-1-yl]-1,4-diethyl-1H-1,2,4-triazol-4-iums 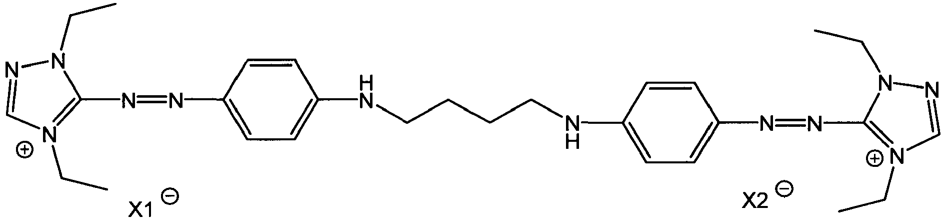
- Salzen des 5-[2-(4-{[5-({4-[2-(1,4-Diethy)-1H-1,2,4-triazol-4-ium-5-yl)diazen-1-yl]phenyl}amino)pentyl]amino}phenyl)diazen-1-yl]-1,4-diethyl-1H-1,2,4-triazol-4-iums 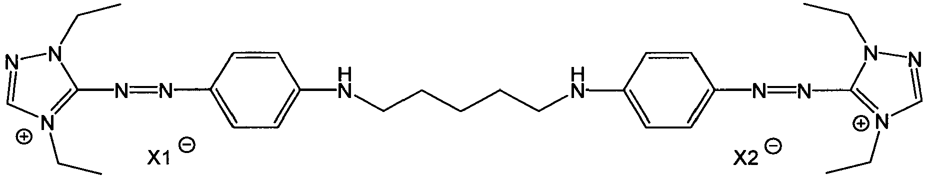
- Salzen des 5-[2-(4-{[3-({4-[2-(1,4-Diethyl-1H-1,2,4-triazol-4-ium-5-yl)diazen-1-yl]phenyl}-(methyl)amino)propyl](methyl)amino}phenyl)diazen-1-yl]-1,4-diethyl-1H-1,2,4-triazol-4-iums 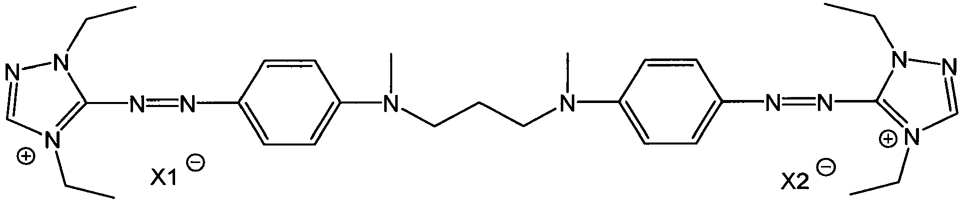
- Salzen des 5-[2-(4-{[4-({4-[2-(1,4-Diethyl-1H-1,2,4-triazol-4-ium-5-yl)diazen-1-yl]phenyl}-(methyl)amino)butyl](methyl)amino}phenyl)diazen-1-yl]-1,4-diethyl-1H-1,2,4-triazol-4-iums 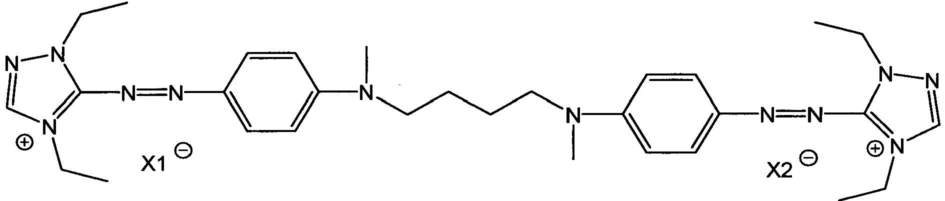
- Salzen des 5-[2-(4-{[5-({4-[2-(1,4-Diethyl-1H-1,2,4-triazol-4-ium-5-yl)diazen-1-yl]phenyl}-(methyl)amino)pentyl](methyl)amino}phenyl)diazen-1-yl]-1,4-diethyl-1H-1,2,4-triazol-4-iums 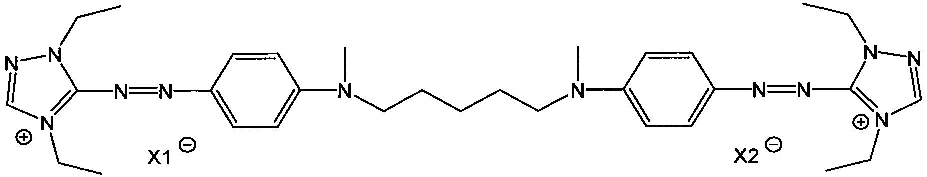
- Salzen des 5-[2-(4-{[3-({4-[2-(1,4-Diethyl-1H-1,2,4-triazol-4-ium-5-yl)diazen-1-yl]phenyl}-(ethyl)amino)propyl](ethyl)amino}phenyl)diazen-1-yl]-1,4-diethyl-1H-1,2,4-triazol-4-iums 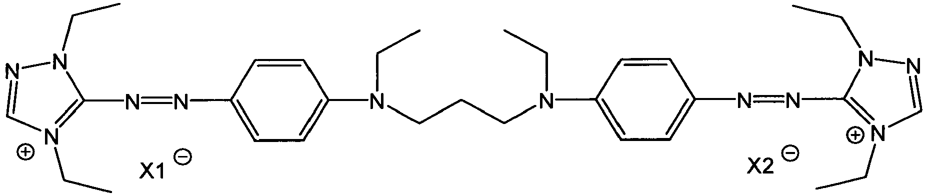
- Salzen des 5-[2-(4-{[4-({4-[2-(1,4-Diethyl-1H-1,2,4-triazol-4-ium-5-yl)diazen-1-yl]phenyl}-(ethyl)amino)butyl](ethyl)amino}phenyl)diazen-1-yl]-1,4-diethyl-1H-1,2,4-triazol-4-iums 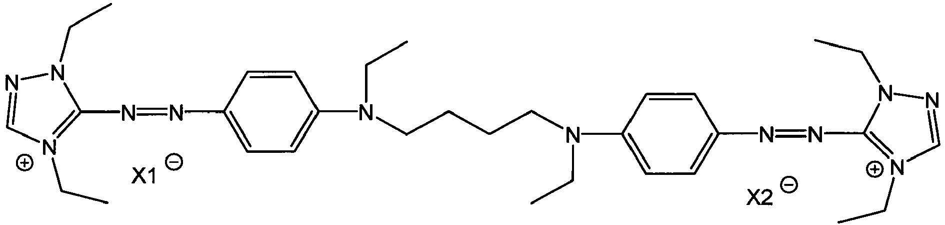
- Salzen des 5-[2-(4-{[5-({4-[2-(1,4-Diethyl-1H-1,2,4-triazol-4-ium-5-yl)diazen-1-yl]phenyl}-(ethyl)amino)pentyl](ethyl)amino}phenyl)diazen-1-yl]-1,4-diethyl-1H-1,2,4-triazol-4-iums

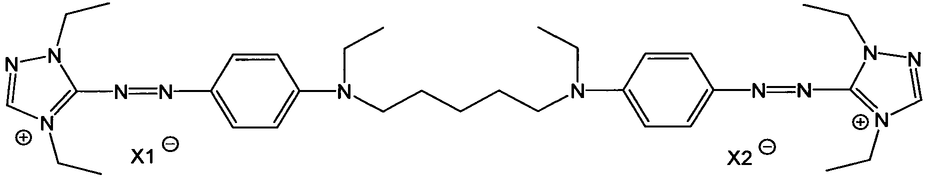

Bei den vorgenannten Verbindungen handelt es sich um dikationische dimere Farbstoffe, wobei das organische Dikation durch die beiden Anionen X1- und X2- neutralisiert wird. Bei den Anionen X1- und X2 handelt es sich jeweils um ein physiologisch verträgliches Anion, bevorzugt aus der Gruppe aus Chlorid, Bromid, lodid, Methylsulfat, Methylsulfonat, p-Toluolsulfonat, Acetat, Hydrogensulfat, ½ Sulfat oder ½ Tetrachlorozinkat. 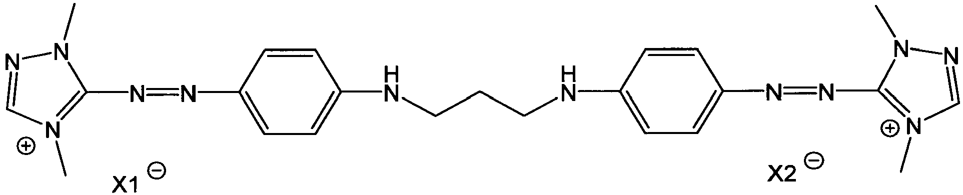 5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Dichlorid
5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Dibromid
5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Sulfat
5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Di(toluolsulfonat)
5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Di(methylsulfat)
5-[2-(4-{[3-({4-[2-(1,4-Dimethyl-1H-1,2,4-triazol-4-ium-5-yl)diazen-1-yl]phenyl}amino)propyl]-amino}phenyl)diazen-1-yl]-1,4-dimethyl-1H-1,2,4-triazol-4-ium Tetrachlorzinkat 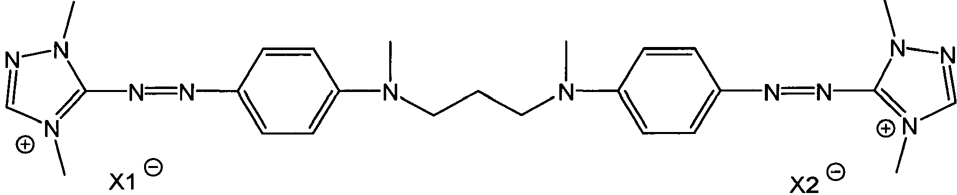 5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Dichlorid
5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Dibromid
5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Sulfat
5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Di(toluolsulfonat)
5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Di(methylsulfat)
5-[2-(4-{[3-({4-[2-(1,4-Dimethyl-1H-1,2,4-triazol-4-ium-5-yl)diazen-1-yl]phenyl}(methyl)amino)propyl]-(methyl)amino}phenyl)diazen-1-yl]-1,4-dimethyl-1H-1,2,4-triazol-4-ium Tetrachlorzinkat.

Die erfindungsgemäßen Mittel zum Färben von keratinischen Fasern enthalten den oder die direktziehenden Farbstoffe der Formel (la) vorzugsweise in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält. Die Mengenangabe in Gewichtsprozent bezieht sich hierbei auf die Gesamtmenge aller im Mittel enthaltenen Verbindungen der Formel (la), die zum Gesamtgewicht des Mittels in Relation gesetzt wird.

In einer weiteren bevorzugten Ausführungsform ist ein erfindungsgemäßes Mittel zum Färben von keratinischen Fasern daher dadurch gekennzeichnet, dass es - bezogen auf das Gesamtgewicht des Mittels - einen oder mehrere direktziehende Farbstoffe (a) der Formel (la) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

Die Farbstoffe der allgemeinen Formel (Ia) können beispielsweise analog zu Verfahren hergestellt werden, wie es in WO 2002/100369 A2 beschrieben ist.

So kann beispielsweise das Edukt 3-Amino-1,2,4-triazol in konzentrierter Schwefelsäure mit Nitrosylschwefelsäure in das Diazoniumion überführt werden:

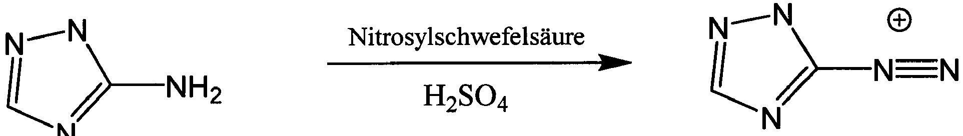

Das reaktive Diazoniumion geht dann mit dimeren Anilin-Derivaten eine doppelte Azokupplungsreaktion ein:

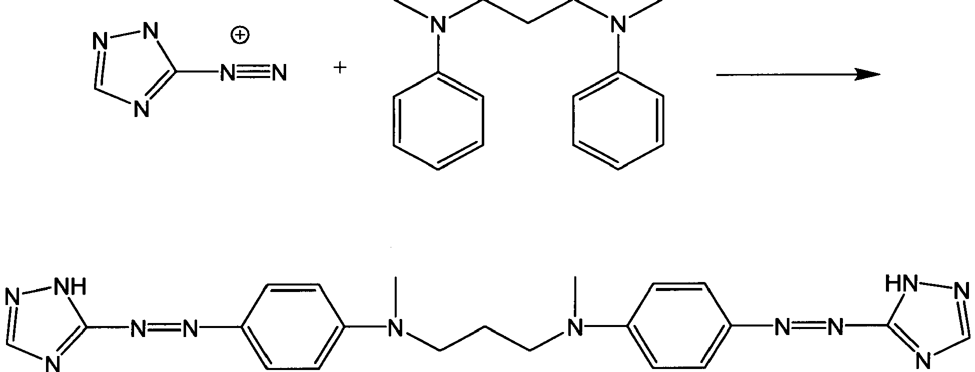

Der in der Azokupplungsreaktion entstehende neutrale dimere Farbstoff kann dann schließlich mit Quaternierungsmitteln quaterniert und so in die dikationische Form überführt werden. Die Quaternierungsreaktion wird bevorzugt in einem polaren aprotischen Lösungsmittel (wie beispielsweise DMSO, DMF etc.) durchgeführt. Als Quaternierungsmittel kommen zum Beispiel Dimethylsulfat, Methylbromid oder p-Toluolsulfonat in Frage.

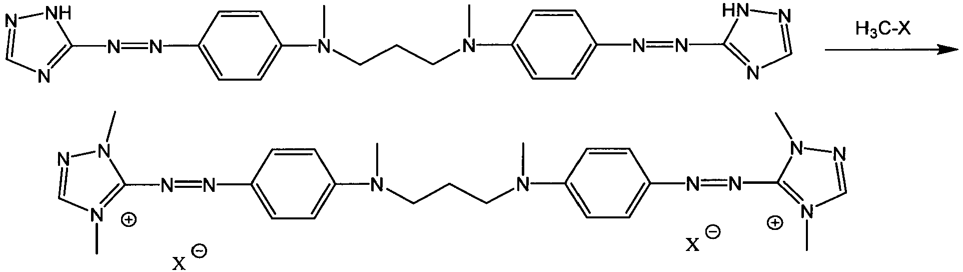

Als zweite erfindungswesentliche Komponente (b) enthalten die Mittel zum Färben von keratinischen Fasern mindestens ein anionisches Tensid und/oder mindestens ein kationisches Tensid

Tenside sind amphiphile (bifunktionelle) Verbindungen, die aus mindestens einem hydrophoben und mindestens einem hydrophilen Molekülteil bestehen. Bei dem hydrophoben Rest handelt es sich bevorzugt eine Kohlenwasserstoffkette mit 8-24 Kohlenstoff-Atomen, die gesättigt oder ungesättigt, linear oder verzweigt sein kann. Besonders bevorzugt ist diese C₈-C₂₄-Alkylkette linear.

Bei anionischen Tensiden umfasst der hydrophile Molekülteil eine negativ geladene hydrophile Kopfgruppe. Bei der negativ geladenen hydrophilen Kopfgruppe kann es sich beispielsweise um eine Carbonsäuregruppe bwz. das Salz einer Carbonsäuregruppe, eine Sulfonsäuregruppe bzw. das Salz der Sulfonsäuregruppe, eine Schwefelsäureestergruppierung bzw. das Salz hiervon, eine Phosphonsäuregruppe bzw. das Salz der Phosphonsäuregruppe, oder eine Phosphorsäureestergruppierung bzw. das Salz hiervon handeln.

Üblicherweise umfasst das erfindungsgemäße kosmetische Mittel einen wässrigen Träger. In wässriger Lösung liegen die vorgenannten hydrophilen Kopfgruppen des anionischen Tensids - wie beispielsweise die Carbonsäure und die Salze der Carbonsäuren - in einem Gleichgewicht vor, dessen Lage vom pH-Wert des Mittels mitbestimmt wird. Wird als anionisches Tensid somit beispielsweise eine Fettsäure eingesetzt, so liegt ein geringer Teil der Fettsäure in wässriger Lösung in Form der protonierten Fettsäure vor, wohingegen der Großteil der Fettsäure in wässriger Lösung deprotoniert und auf diesem Wege in das Salz der Fettsäure überführt wird. Aus diesem Grund umfasst die Definition eines anionischen Tensid auch ein Tenside mit einer - noch protonierten - Säuregruppe.

Ein anionisches Tensid (b) im Sinne der vorliegenden Erfindung enthält keine kationischen Gruppierungen, d.h. zwitterionische Tenside sind von der Definition eines anionischen Tensids nicht mit umfasst.

Erfindungsgemäße anionische Tenside sind demnach gekennzeichnet durch die Anwesenheit einer wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein.

Typische Beispiele für anionische Tenside sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Beispiele für erfindungsgemäße anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen. Sulfobernsteinsäuremono- und -dialkylester können durch Umsetzung von Maleinsäureanhydrid mit einem Fettalkohol mit 8 - 24 C-Atomen zum Maleinsäuremonoester des Fettalkohols und Weiterreaktion mit Natriumsulfit zum Sulfobernsteinsäureester hergestellt werden. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren Mischungen sowie deren salzen, CH₃-(CH₂)_{y}-CHOH-(CH₂)ₚ-(CH-SO₃M)-(CH₂)_{z}-CH₂-O-(CₙH₂ₙO)ₓ-H, und/oder CH₃-(CH₂)_{y}-(CH-SO₃M)-(CH₂)ₚ-CHOH-(CH₂)_{z}-CH₂-O-(CₙH₂ₙO)ₓ-H wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel R¹-(CHOSO₃M)-CHR³-(OCHR⁴-CH₂)n-OR² mit R¹, einem linearen Alkylrest mit 1 bis 24 C-Atomen, R² für einen linearen oder verzweigten, gesättigten Alkylrest mit 1 bis 24 C-Atomen, R³ für Wasserstoff oder einen linearen Alkylrest mit 1 bis 24 C-Atomen, R⁴ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in R¹ und R³ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, R¹(OCH₂CH₂)ₙ-O-(PO-OX)-OR²,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel RCO(AlkO)ₙSO₃M
   in der RCO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie ⁺NR³R⁴R⁵R⁶, mit R³ bis R⁶ unabhängig voneinander stehend für Wasserstoff oder einen C1 bis C4 - Kohlenwasserstoffrest,
- Monoglyceridsulfate und Monoglyceridethersulfate der Formel R⁸OC-(OCH₂CH₂)ₓ-OCH₂-[CHO(CH₂CH₂O)_{y}H]-CH₂O(CH₂CH₂O)_{z}-SO₃X,
   in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der R⁸CO für einen linearen Acylrest mit 8 bis 18 Kohlenstoffatomen steht,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

Die Behandlung von keratinischen Fasern mit Mitteln, die (a) mindestens einen direktziehenden Farbstoff der Formel (Ia) und (b) mindestens ein anionisches Tensid enthielten, führte zu intensiven Färbungen in attraktiven, reinen Blaunuancen ohne Rotanteil. Hierbei hat sich überraschenderweise gezeigt, dass das Farbaufzugsvermögen durch Einsatz eines oder mehrere spezieller anionischer Tenside noch weiter optimiert werden konnte. Besonders intensive Blaufärbungen wurden erhalten, wenn die Farbstoffe (a) der Formel (Ia) mit mindestens einem anionischen Tensid (b) aus der Gruppe der
- linearen und verzweigten Fettsäuren mit 8 bis 30 C-Atomen,
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von Fettsäuren mit dem Natriumsalz der 2-Hydroxyethan-sulfonsäure (Isethionsäure) zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo® erhältlich, und
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht.

In einer besonders bevorzugten Ausführungsform ist ein erfindungsgemäßes Mittel daher dadurch gekennzeichnet, dass es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
   - R: für eine lineare Alkylgruppe mit 8 bis 30 C-Atomen steht,
   - x: für eine ganze Zahl von 0 bis 16 steht,
   - M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
   - R¹: für eine C2-C30-Alkylgruppe steht,
   - y: für eine ganze Zahl von 1 bis 20 steht,
   - R²: für Wasserstoff, einen Methyl-, Ethyl-, Propyl-, Isopropyl-, n-Butyl-, t-Butyl- oder iso-Butylrest steht, und
   - M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,

Ein besonders gutes Farbaufzugsvermögen konnte beobachtet werden, wenn als Aniontensid (b) mindestens eine Ethercarbonsäuren der Formel R-O-(CH2-CH2O)x-CH2-COOH oder ihr Salz eingesetzt wurde. Der Einsatz von - gegebenenfalls ethoxylierten - Ethercarbonsäuren ist daher explizit ganz besonders bevorzgut.

In einer explizit ganz besonders bevorzugten Ausführungsform ist ein erfindungsgemäßes Mittel daher dadurch gekennzeichnet, dass als anionisches Tensid (b) mindestens eine Ethercarbonsäure der Formel (B1) enthält

R-O-(CH2-CH2O)x-CH2-COOM (B1),

wobei
- R: für eine lineare Alkylgruppe mit 8 bis 30 C-Atomen steht,
- x: für eine ganze Zahl von 0 bis 16 steht,
- M: für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht.

Weiterhin besonders bevorzugt steht x für eine ganze Zahl von 5 bis 11, ganz besonders bevorzugt für die Zahlen 5, 6 oder 7.

In einer weiterhin ganz besonders bevorzugten Ausführungsform ist ein erfindungsgemäßes Mittel daher dadurch gekennzeichnet, dass als anionisches Tensid (b) mindestens eine Ethercarbonsäure der Formel (B1) enthält

R-O-(CH2-CH2O)x-CH2-COOM (B1),

wobei
- R: für eine lineare Alkylgruppe mit 8 bis 30 C-Atomen steht,
- x: für eine ganze Zahl von 5 bis 11 steht,
- M: für Wasserstoff oder ein Metall wie Alkalimetall, insbesondere Natrium, Kalium, Lithium, Erdalkalimetall, insbesondere Magnesium, Calcium, Zink, oder ein Ammoniumion (NH4⁺) steht.

Die ganz besonders bevorzugten anionischen Tenside der Formel (B1) sind beispielsweise unter dem Handelsnamen erhältlich
- Akypo Soft 45 HP der Firma Kao (Natrium Laureth-6 Carboxylat)
- Akypo Soft 45 NV der Firma Kao (Natrium Laureth-5 Carboxylat)
- Akypo RLM 100 NV der Firma Kao (Natrium Laureth-11 Carboxylat)

Bevorzugt enthalten die erfindungsgemäßen Mittel zum Färben von keratinischen Fasern ein oder mehrere anionische Tenside (b) in einer Gesamtmenge von 0,05 bis 4,5 Gew.-%, bevorzugt von 0,1 bis 2,1 Gew.-%, weiter bevorzugt von 0,15 bis 1,8 Gew.-% und ganz besonders bevorzugt von 0,2 bis 0,9 Gew.-%. Hierbei sind die Angaben in Gew.-% auf die Gesamtmenge aller anionischen Tenside (b) bezogen, die zur Gesamtmenge des Färbemittels in Relation gesetzt werden.

In einer weiteren besonders bevorzugten Ausführungsform ist ein erfindungsgemäßes Mittel zum Färben von keratinischen Fasern daher dadurch gekennzeichnet, dass es - bezogen auf das Gesamtgewicht des Mittels - ein oder mehrere anionische Tenside (b) in einer Gesamtmenge von 0,05 bis 4,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

Wie zuvor beschrieben hat sich gezeigt, dass durch Variation des bzw. der anionischen Tenside (b) das Farbaufzugsvermögen der direktziehenden Farbstoffe der Formel (la) beeinflusst werden kann.

Generell kann der Farbaufzug durch Einsatz mindestens eines anionischen Tensids verbessert werden. Der Einsatz mindestens einer - gegebenenfalls ethoxylierten - Ethercarbonsäure und/oder ihres Salzes, wie sie in Formel (B1) beschrieben ist, hat sich in diesem Zusammenhang als besonders vorteilhaft erwiesen. In diesem Zusammenhang wurde beobachtet, dass die Keratinfasern sich insbesondere dann in intensiven Rotnuancen färben ließen, wenn das bzw. die Ethercarbonsäuren (und/oder ihre Salze) als das hauptsächliche anionische Tensid im erfindungsgemäßen Mittel enthalten waren.

Mit anderen Worten waren die Färbeergebnisse dann besonders intensiv, wenn die erfindungsgemäßen Mitteln ein oder mehrere Ethercarbonsäuren der Formel (B1) enthielten, und wenn darüber hinaus alle weiteren eingesetzten anionischen Tenside nur in geringeren Mengen vorhanden waren. Der Einsatz der Ethercarbonsäure(n) der Formel (B1) als Haupt-Tensid lässt sich durch Angabe eines Gewichtsverhältnisses quantifizieren, welches die Gesamtmenge der im Mittel enthaltenen anionischen Tenside der Formel (B1) zur Gesamtmenge aller im Mittel enthaltenen anionischen Tenside (b) in Relation setzt.

In einer weiteren besonders bevorzugten Ausführungsform ist ein erfindungsgemäßes Mittel zum Färben von keratinischen Fasern daher dadurch gekennzeichnet, dass das Gewichtsverhältnis aus allen im Mittel enthaltenen anionischen Tensiden der Formel (B1) zur Gesamtmenge der im Mittel enthaltenen anionischen Tenside bei einem Wert von 0,5, bevorzugt von 0,6, weiter bevorzugt von 0,75 und besonders bevorzugt von 0,9 liegt.

Beispiel: Ein Mittel zum Färben von keratinischen Fasern enthält:
(a) 1,0 g 5-[2-(4-{[3-({4-[2-(1,4-Dimethyl-1H-1,2,4-triazol-4-ium-5-yl)diazen-1-yl]phenyl}(methyl)amino)-propyl](methyl)amino}phenyl)diazen-1-yl]-1,4-dimethyl-1H-1,2,4-triazol-4-ium Tetrachlorzinkat
(b) 0,23 g Natrium laureth-6 carboxylat (B1) und
(b) 0,10 g Natriumlaureth-Sulfat (2 EO)

Das Gewichtsverhältnis aus allen im Mittel enthaltenen anionischen Tensiden der Formel (B1) zur Gesamtmenge der im Mittel enthaltenen anionischen Tenside liegt bei einem Wert von [0,23 / (0,23 + 0,1)] = 0,69

Beispiel: Ein Mittel zum Färben von keratinischen Fasern enthält:
(a) 1,0 g 5-[2-(4-{[3-({4-[2-(1,4-Dimethyl-1H-1,2,4-triazol-4-ium-5-yl)diazen-1-yl]phenyl}(methyl)amino)-propyl](methyl)amino}phenyl)diazen-1-yl]-1,4-dimethyl-1H-1,2,4-triazol-4-ium Di(methylsulfat)
(b) 0,23 g Natrium laureth-6 carboxylat (B1)

Das Gewichtsverhältnis aus allen im Mittel enthaltenen anionischen Tensiden der Formel (B1) zur Gesamtmenge der im Mittel enthaltenen anionischen Tenside liegt bei einem Wert von [0,23 / 0,23] = 1,0

Bei der zweiten erfindungswesentlichen Komponente (b) kann es sich auch um ein kationisches Tensid handeln. Bei Applikation der erfindungsgemäßen Farbstoffe der Formel (la) in Kombination mit mindestens einem kationischen Tensid konnten ebenfalls Färbungen mit stark verbessertem Farbaufzug und erhöhter Farbintensität erzielt werden.

Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren und Grenzflächen und aggregieren in wässriger Lösung oberhalb der kritischen Micellbildungskonzentration zu positiv geladenen Micellen.

Beispiele für Kationentenside sind
- quartäre Ammniumverbindungen, die als hydrophobe Reste ein oder zwei Alkylketten mit einer Kettenlänge von 8 bis 28 C-Atomen tragen können
- quartäre Phosphoniumsalze, substituert mit einer oder mehrern Alkylketten mit einer einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, diese ist beispielsweise bei Esterquats der Fall.

Innerhalb der Gruppe der kationischen Tenside (b) konnte insbesondere mit den kationischen Ammoniumverbindungen der Formel (B2) ein verbesserter Farbaufzug und damit verbunden eine Erhöhung der Farbintensität erzielt werden. Die kationischenTenside der Formel (B2) sind daher besonders bevorzugt, 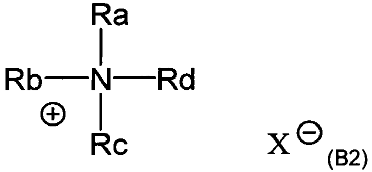 worin
- Ra, Rb: unabhängig voneinander für eine C₁-C₆-Alkylgruppe, eine C₂-C₆-Alkenylgruppe oder eine C₂-C₆-Hydroxyalkylgruppe stehen,
- Rc: für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
- Rd: für eine C₈-C₂₈-Alkylgruppe steht und
- X-: für ein physiologisch verträgliches Anion steht.

In einer weiteren bevorzugten Ausführungsform ist ein erfindungsgemäßes Produkt daher dadurch gekennzeichnet, dass es als kationische(s) Tensid(e) (c) eine oder mehrere Verbindungen der Formel (B2) enthält, 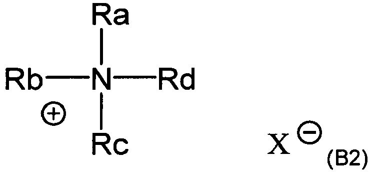 worin
- Ra, Rb: unabhängig voneinander für eine C₁-C₆-Alkylgruppe, eine C₂-C₆-Alkenylgruppe oder eine C₂-C₆-Hydroxyalkylgruppe stehen,
- Rc: für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
- Rd: für eine C₈-C₂₈-Alkylgruppe steht und
- X-: für ein physiologisch verträgliches Anion steht.

Die Substituenten Ra, Rb, Rc, und Rd der Verbindung der Formeln (B2) sind nachstehend beispielhaft erläutert: Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt.

Bevorzugt stehen Ra und Rb unabhängig voneinander für eine C₁-C₆-Alkylgruppe. Besonders bevorzugt ist es, wenn Ra und Rb unabhängig voneinander für eine Methylgruppe, eine Ethylgruppe oder eine n-Propylgruppe stehen. In einer ganz besonders bevorzugten Ausführungsform stehen Ra und Rb beide für eine Methylgruppe oder beide für eine Ethylgruppe.

Rd und gegebenenfalls Rc stehen für eine C₈-C₂₈-Alkylgruppe. Diese Alkylgruppen können verzweigt oder unverzweigt, gesättigt oder ungesättigt sein. Wenn die C₈-C₂₈-Alkylgruppe ungesättigt ist, kann sie ein oder mehrfach ungesättigt sein, d.h. ein oder mehrere Doppelbindungen besitzen.

Bevorzugt steht Rc für eine C₁-C₇-Alkylgruppe. Ganz besonders bevorzugt steht Rc für eine Methylgruppe, eine Ethylgruppe oder eine Propylgruppe, insbesondere für eine Methylgruppe.

Wenn Rd für eine lineare, gesättigte C₁₆-C₂₂-Alkylgruppe steht, wirkt sich dies ganz besonders vorteilhaft auf die Eigenschaften des durch Versprühen entstehenden Schaums aus. Bevorzugt steht R4 daher für eine lineare, gesättigte C₁₆-C₂₂-Alkylgruppe. Ganz besonders bevorzugt ist es, wenn R4 für eine lineare, gesättigte C₂₀-Alkylgruppe oder eine lineare, gesättigte C₂₂-Alkylgruppe steht.

In einer weiteren ganz besonders bevorzugten Ausführungsform ist ein erfindungsgemäßes Produkt daher dadurch gekennzeichnet, dass es als kationische(s) Tensid(e) (c) ein oder mehrere Verbindungen der Formel (B2) enthält, 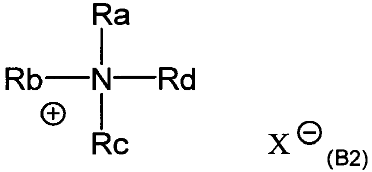 worin
- Ra, Rb: unabhängig voneinander für eine C₁-C₆-Alkylgruppe, bevorzugt beide für eine Methylgruppe, stehen,
- Rc: für eine Methylgruppe, eine Ethylgruppe oder eine Propylgruppe, bevorzugt für eine Methylgruppe, steht, und
- Rd: für eine C₁₆-C₂₂-Alkylgruppe, bevorzugt für eine C₂₀-Alkylgruppe oder eine C₂₂-Alkylgruppe,
- X-: für ein physiologisch verträgliches Anion steht.

Bevorzugte kationische Tenside der Formel (B2) sind beispielsweise physiologisch verträgliche Salze des N,N,N-Trimethyl-1-hexadecanaminiums, insbesondere N,N,N-Trimethyl-1-hexadecanaminiumchlorid, welches auch unter dem Handelsnamen Dehyquart A-CA vertrieben wird. Ebenfalls bevorzugt sind Salze des Trimethylstearylammoniums, insbesondere Trimethylstearylammoniumchlorid, welches auch unter dem Handelsnamen Genamin STAC kommerziell erhältlich ist.

Weitere besonders bevorzugte kationische Tenside der Formel (B2) sind Salze des Trimethyl-1-eicosanaminiums, insbesondere Trimethyl-1-eicosanaminiumchlorid, und Salze des Trimethyl-1-docosanaminiums, insbesondere Trimethyl-1-docosanaminiumchlorids. Ein Gemisch beider Verbindungen ist unter dem Handelsnamen Genamin KDMP von der Firma Clariant erhältlich.

X⁻ steht in den Fomel (B2) jfür ein physiologisch verträgliches Anion. Geeignete physiologisch verträgliche Anionen sind Halogenid, Hydrogensulfat, Sulfat, Benzolsulfonat, p-Toluolsulfonat, Acetat, Citrat, Lactat, Tartrat, Methylsulfat (H₃COSO₃⁻), Methylsulfoant oder Trifluormethansulfonat. Besonders bevorzugt steht A- für Chlorid, Bromid oder für Methylsulfat (H₃COSO₃⁻).

Weitere kationische Tenside können aus der Gruppe der kationischen Imidazoliumverbindungen ausgewählt werden.

Das erfindungsgemäße Mittel kann das bzw. die kationischen Tenside in einer Gesamtmenge von 0,1 bis 4,8 Gew.-%, bevorzugt von 0,2 bis 2,4 Gew.-%, weiter bevorzugt von 0,3 bis 1,8 Gew.-%- bezogen auf das Gesamtgewicht des Mittels - enthalten.

In einer weiteren bevorzugten Ausführungsform ist ein erfindungsgemäßes Produkt daher dadurch gekennzeichnet, dass ein oder mehrere kationische Tenside (b) in einer Gesamtmenge von 0,1 bis 4,8 Gew.-%, bevorzugt von 0,2 bis 2,4 Gew.-%, weiter bevorzugt von 0,3 bis 1,8 Gew.-%- bezogen auf das Gesamtgewicht des Mittels - enthält.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

In einer weiteren bevorzugten Ausführungsform enthalten die erfindungsgemäßen Mittel neben der Verbindung der Formel (la) zusätzlich mindestens einen weiteren direktziehenden Farbstoff. Durch die Kombination mit weiteren kationischen direktziehenden Farbstoffen lässt sich das erzielbare Nuancenspektrum erweitern, und die Färbeeigenschaften lassen sich noch weiter verbessern. Direktziehende Farbstoffe können in anionische, kationische und nichtionische direktziehende Farbstoffe unterteilt werden.

Die direktziehenden Farbstoffe sind vorzugsweise ausgewählt aus den kationischen direktziehenden Farbstoffen, da diese mit den Farbstoffen der allgemeinen Formel (la) gut kompatibel sind.

In einer weiteren besonders bevorzugten Ausführungsform ist ein erfindungsgemäßes Mittel dadurch gekennzeichnet, dass es zusätzlich mindestens einen weiteren kationischen direktziehenden Farbstoff enthält, der von den Farbstoffen der Formel (la) verschieden ist.

Als besonders gut kompatibel haben sich ein oder mehrere Farbstoffe aus der Gruppe Basic Yellow 87, Basic Orange 31, Basic Red 51, Basic Violet 2 und Cationic Blue 347 erwiesen.

Ganz besonders gut kompatibel sind die Farbstoffe der Formel (la) mit den kationischen Azofarbstoffen Basic Orange 31 und Basic Red 51.

In einer weiteren besonders bevorzugten Ausführungsform ist ein erfindungsgemäßes Mittel dadurch gekennzeichnet, dass es zusätzlich Basic Orange 31 und/oder Basic Red 51 enthält.

Das erfindungsgemäße Mittel kann aber auch zusätzlich mindestens einen nichtionischen direktziehenden Farbstoff enthalten. Dieser kann ausgewählt sein aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind. Die erfindungsgemäßen Mittel können weiterhin auch zusammen mit Oxidationsfärbemitteln eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die zusätzlichen direktziehenden Farbstoffe, Entwicklerkomponenten und Kupplerkomponenten werden bevorzugt jeweils in einem Anteil von 0,0001 bis 5,0 Gew.-%, vorzugsweise 0,001 bis 3,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Soll die Färbung mit den erfindungsgemäßen direktziehenden Farbstoffen der Formel (I) und eine oxidative Aufhellung der Keratinfasern in einem Schritt erfolgen, so enthalten die erfindungsgemäßen Mittel zusätzlich ein Oxidationsmittel, bevorzugt Wasserstoffperoxid und/oder eines seiner festen Anlagerungsprodukte an organische oder anorganische Verbindungen.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von. den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

In einer weiteren besonders bevorzugten Ausführungsform ist ein erfindungsgemäßes Mittel dadurch gekennzeichnet, dass es - bezogen auf das Gesamtgewicht des Mittels - 0,5 bis 12,5 Gew.-%, bevorzugt 2,5 bis 10 Gew.-% und insbesondere 3 bis 6 Gew.-% Wasserstoffperoxid enthält.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

In einer weiteren besonders bevorzugten Ausführungsform ist ein erfindungsgemäßes Mittel dadurch gekennzeichnet, dass es zusätzlich mindestens ein Persulfat aus der Gruppe Ammoniumperoxodisulfat, Kaliumperoxodisulfat und Natriumperoxodisulfat enthält.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Das Färbe- und/oder Mattier-Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Die Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Die erfindungsgemäßen Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Mit den Färbemitteln, welche die erfindungsgemäßen direktziehenden Farbstoffe der allgemeinen Formel (la) enthalten, lassen sich keratinische Fasern in ausgesprochen attraktiven und intensiven Rottönen färben. Bei den Farbstoffen der allgemeinen Formel (la) handelt es sich um kationische dimere Azofarbstoffe. Hierbei hat sich überraschenderweise herausgestellt, dass die Farbintensität von kationischen Azofarbstoffen durch Zusatz eines oder mehrere anionischer Tenside (b) der Formel (B1) noch weiter gesteigert werden kann,

R-O-(CH2-CH2O)x-CH2-COOM (B1),

wobei
- R: für eine lineare Alkylgruppe mit 8 bis 30 C-Atomen steht,
- x: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Ein weiterer Gegenstand der vorliegenden Erfindung ist daher die Verwendung von einem oder mehreren anionischen Tensiden (b) der Formel (B1)

R-O-(CH2-CH2O)x-CH2-COOM (B1),

wobei
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht, zur Verbesserung des Farbaufzugsvermögens von direktziehenden Farbstoffen der allgemeinen Formel (la), wie sie bei der vorangegangenen Beschreibung des ersten Erfindungsgegenstandes offenbart sind, auf keratinischen Fasern.

Unter der Verbesserung des Farbaufzugsvermögens wird in diesem Zusammenhang verstanden, dass die Farbstoffe vermehrt bzw. verstärkt in die keratinische Faser hineindiffundieren, was zu Färbungen mit höherer Farbintensität führt. Die verstärkte Farbintensität lässt sich enweder visuell durch Betrachtung unter einer Tageslichtlampe oder aber durch farbmetrische Vermessung (Bestimmung der Lab-Werte) detektieren.

Überraschenderweise hat sich weiterhin herausgestellt, dass die Farbintensität von kationischen Azofarbstoffen auch durch Zusatz eines oder mehrere katonischer Tenside (b) der Formel (B2) gesteigert werden kann, 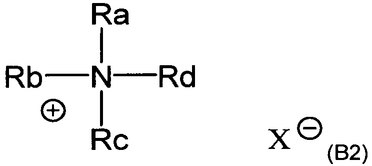 worin
- Ra, Rb: unabhängig voneinander für eine C₁-C₆-Alkylgruppe, eine C₂-C₆-Alkenylgruppe oder eine C₂-C₆-Hydroxyalkylgruppe stehen,
- Rc: für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
- Rd: für eine C₈-C₂₈-Alkylgruppe steht und
- X-: für ein physiologisch verträgliches Anion steht.

Ein weiterer Gegenstand der vorliegenden Erfindung ist daher die Verwendung von einem oder mehreren katonischen Tensiden (b) der Formel (B2), 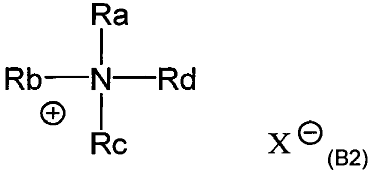 worin
- Ra, Rb: unabhängig voneinander für eine C₁-C₆-Alkylgruppe, eine C₂-C₆-Alkenylgruppe oder eine C₂-C₆-Hydroxyalkylgruppe stehen,
- Rc: für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
- Rd: für eine C₈-C₂₈-Alkylgruppe steht und
- X-: für ein physiologisch verträgliches Anion steht.
zur Verbesserung des Farbaufzugsvermögens von direktziehenden Farbstoffen, wie sie bei der vorangegangenen Beschreibung des ersten Erfindungsgegenstandes offenbart sind, auf keratinischen Fasern.

Die Mittel des ersten Erfindungsgegenstands können in Verfahren zum Färben und auch in Verfahren zum gleichzeitigen Blondieren bzw. Aufhellen und Färben menschlicher Haare genutzt werden.

Die erfindungsgemäßen Mittel können als Einkomponentenmittel oder als Mehrkomponentenmittel wie Zweikomponentenmittel oder Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt. Unter dem erfindungsgemäßen Mittel zur Farbveränderung von keratinischen Fasern wird immer das anwendungsbereite Mittel verstanden.

Wird das erfindungsgemäße Mittel dem Anwender in Form eines Einkomponentenmittels zur Verfügung gestellt, dann muss das anwendungsbereite Mittel nicht erst hergestellt werden, sondern kann direkt aus dem Behältnis, in dem es konfektioniert wurde, entnommen und auf die keratinischen Fasern appliziert werden.

Bei Blondiermitteln handelt es sich jedoch üblicherweise um Zweikomponentenprodukte, bei welchen eine oxidationsmittelhaltige Komponente (A1) kurz vor der Anwendung mit einem (Alkalisierungs-)Mittel (A2) vermischt und diese anwendungsbereite Mischung auf die Haare aufgetragen wird.

In diesem Fall handelt es sich bei dem erfindungsgemäßen Mittel um das anwendungsbereite Mittel, welches kurz vor der Anwendung durch Vermischen von (A1) und (A2) hergestellt wurde.

Hierbei können die direktziehenden Farbstoffe der allgemeinen Formel (I) in der Komponente (A1) (d.h. zusammen mit dem Oxidationsmittel) oder aber in der Komponente (A2) (zusammen mit dem Alkalisierungsmittel) konfektioniert werden. Das anionische und/oder kationische Tensid (b) kann hierbei in der Komponente (A1) und/oder in der Komponente (A2) enthalten sein.

Es ist ebenfalls möglich und erfindungsgemäß, wenn das anwendungsbereite Mittel kurz vor der Anwendung auf den menschlichen Haaren durch Vermischen von 3 Komponenten hergestellt wird, wobei
- die Komponente (A1) mindestens einen direktziehenden Farbstoff der allgemeinen Formel (I) und mindestens ein Alkalisierungsmittel
- die Komponente (A2) mindestens ein erstes Oxidationsmittel (z.B. Wasserstoffperoxid) und
- die Komponente (A3) mindestens ein zweites Oxidationsmittel (z.B. ein oder mehrere Poroxodisulfatsalze) enthält. Das anionische und/oder kationische Tensid (b) kann hierbei in der Komponente (A1) und/oder in der Komponente (A2) und/oder in der Komponente (A3) enthalten sein.

Während der Einwirkzeit der Mittel auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die behandelte Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Bezüglich weiterer bevorzugter Ausführungsformen der erfindungsgemäßen Verwendung und Verfahren gilt mutatis mutandis das zu den erfindungsgemäßen Mitteln Gesagte.

### Beispiele

### Direkzieher 1: 5-[2-(4-{[3-({4-[2-(1,4-Dimethyl-1H-1,2,4-triazol-4-ium-5-yl)diazen-1-yl]phenyl}-(methyl)amino)propyl](methyl)amino}phenyl)diazen-1-yl]-1,4-dimethyl-1H-1,2,4-triazol-4-ium Di(methylsulfat)

Der Farbstoff DZ 1 wurde in Analogie zu einem Verfahren synthetisiert, wie es in den Dokumenten WO 2002/100369 A2 und US 3291788 beschrieben ist.

Als Edukte wurden 2-Amino-1,2,4-triazol und N,N'-Dimethyl-N,N'-diphenyl-propan-1,3-diamin eingesetzt (Azokupplungsreaktion in wässriger, schwefelsauerer Lösung mit Nitrosylschwefelsäure). Der in dieser Azokupplungsreaktion entstehende neutrale Farbstoff wurde im Anschluss daran quaterniert (beispielsweise mit dem Quaternierungsmittel Dimethylsulfat in einem polaren, aprotischen Lösungsmittel wie Dimethylformamid oder Dimethylsulfoxid).

### DZ 1 (erfindungsgemäß)

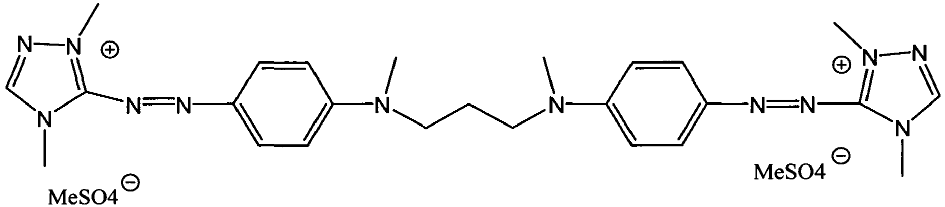

### Färbebeispiele

### Formulierungen

Es wurden die folgenden Färbecremes hergestellt (alle Angaben in Gew.-%, Aktivsubstanz)

| | **V1** | **E1** | **E2** |
|---|---|---|---|
| Cetearyl Alcohol (C16/C18-Fettalkohol) | 1,0 | 1,0 | 1,0 |
| Coconut Alcohol (C12/C18-Fettalkohol) | 1,0 | 1,0 | 1,0 |
| Methylparaben | 0,1 | 0,1 | 0,1 |
| Propylparaben | 0,1 | 0,1 | 0,1 |
| Cocoamidopropylbetain (zwitterionisches Tensid) | 1,0 | --- | --- |
| Natrium Laureth-5 Carboxylat | --- | 1,0 | --- |
| Cetyltrimethylammoniumchlorid | --- | --- | 1,0 |
| DZ 1 (erfindungsgemäß) | 0,5 | 0,5 | 0,5 |
| Ammoniumsulfat | 1,0 | 1,0 | 1,0 |
| Wasser | ad 100 | ad 100 | ad 100 |

Die Fettalkohole (Fettkörper) und die Konservierungsmittel wurden jeweils zusammen mit den Tensiden aufgeschmolzen. Diese Schmelze wurde mit heißem Wasser emulgiert, anschließend wurde der in Propylenglycol vorgelöste Farbstoff hinzu gegeben sowie die Ammoniumsulfatlösung hinzugefügt. Der angegebene pH-Wert wurde mit Ammoniak eingestellt, danach wurde mit Wasser auf 100 g aufgefüllt.

### Ausfärbungen

Jeweils 1,8 g der Färbecreme wurden auf eine ca. 6 cm lange Strähne Menschenhaares (Kerling Euronaturhaar, 80 % ergraut) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Nach dem Trocknen wurden die Färbung und die Farbintensität der Strähnen visuell unter der Tageslichtlampe beurteilt.

| | Formulierung | pH-Wert | Farbnuance | Farbintensität |
|---|---|---|---|---|
| V1 | DZ 1 und Cocoamidopropylbetain | 9,5 | granatrot | +++ |
| E1 | DZ 1 und Natrium Laureth-5 Carboxylat | 9,5 | granatrot | +++++ |
| E2 | DZ 1 und Cetyltrimethylammoniumchlorid | 9,5 | granatrot | +++++ |

| | | | | |
|---|---|---|---|---|
| Farbintensität: + = schlecht +++ = mittel +++++ = sehr gut | | | | |

## Patentansprüche

1. Mittel zum Färben von keratinischen Fasern, insbesondere menschlichen Haaren, enthaltend in einem kosmetischen Träger
(a) mindestens einen direktziehenden Farbstoff der Formel (la), 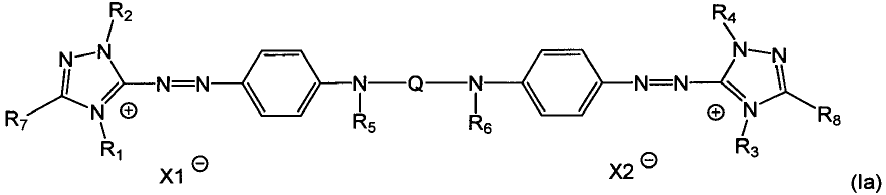 wobei
R1, R2, R3, R4 unabhängig voneinander für eine C₁-C₆-Alkylgruppe, eine C₂-C₆-Alkenylgruppe oder für eine Hydroxy-C₂-C₆-alkylgruppe stehen,
R5, R6 unabhängig voneinander für ein Wasserstoffatom, eine C₁-C₆-Alkylgruppe, eine C₂-C₆-Alkenylgruppe, eine Hydroxy-C₂-C₆-alkylgruppe oder für eine Cyano-C₁-C₆-alkylgruppe stehen,
R7, R8 unabhängig voneinander für ein Wasserstoffatom, eine C₁-C₆-Alkylgruppe, ein Halogenatom aus der Gruppe Chlor, Brom, Fluor oder Jod, oder für eine C₁-C₆-Alkoxygruppe stehen,
X1, X2 unabhängig voneinander für ein physiologisch verträgliches Anion, bevorzugt aus der Gruppe Chlorid, Bromid, lodid, Methylsulfat, Methylsulfonat, p-Toluolsulfonat, Acetat, Hydrogensulfat, ½ Sulfat oder ½ Tetrachlorozinkat, stehen,
Q für eine Gruppierung der Formel (II) steht, 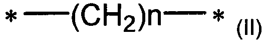
n für eine ganze Zahl von 3 bis 6 steht,
und
(b) mindestens ein anionisches Tensid und/oder mindestens ein kationisches Tensid.

2. Mittel nach Anspruch 1, **dadurch gekennzeichnet, dass** es (a) mindestens einen direktziehenden Farbstoff der allgemeinen Formel (Ia) enthält, in der
R1, R2, R3, R4 unabhängig voneinander für eine Methylgruppe oder für eine Ethylgruppe stehen und
R5, R6 unabhängig voneinander für ein Wasserstoffatom, eine Methylgruppe oder für eine Ethylgruppe stehen.

3. Mittel nach einem der Ansprüche 1 oder 2, **dadurch gekennzeichnet, dass** es (a) mindestens einen direktziehenden Farbstoff der allgemeinen Formel (Ia) enthält, in der
R7, R8 unabhängig voneinander für ein Wasserstoffatom, eine Methylgruppe oder für eine Methoxygruppe, bevorzugt für ein Wasserstoffatom, stehen.

4. Mittel nach einem der Ansprüche 1 bis 3, **dadurch gekennzeichnet, dass** es (a) mindestens einen direktziehenden Farbstoff der allgemeinen Formel (Ia) enthält, der ausgewählt ist aus
- Salzen des 5-[2-(4-{[3-({4-[2-(1,4-Dimethyl-1H-1,2,4-triazol-4-ium-5-yl)diazen-1-yl]phenyl}amino)propyl]amino}phenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]phenyl}amino)butyl]amino}phenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]phenyl}amino)pentyl]amino}phenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]phenyl}-(methyl)amino)propyl](methyl)amino}phenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]phenyl}-(methyl)amino)butyl](methyl)amino}phenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]phenyl}-(methyl)amino)pentyl](methyl)amino}phenyl)diazen-1-yl]-1,4-dimethyl-1H-1,2,4-triazol-4-iums
- Salzen des 5-[2-(4-{[3-({4-[2-(1,4-Dimethyl-1H-1,2,4-triazol-4-ium-5-yl)diazen-1-yl]phenyl}-(ethyl)amino)propyl](ethyl)amino}phenyl)diazen-1-yl]-1,4-dimethyl-1H-1,2,4-triazol-4-iums
- Salzen des 5-[2-(4-{[4-({4-[2-(1,4-Dimethyl-1H-1,2,4-triazol-4-ium-5-yl)diazen-1-yl]phenyl}-(ethyl)amino)butyl](ethyl)amino}phenyl)diazen-1-yl]-1,4-dimethyl-1H-1,2,4-triazol-4-iums
- Salzen des 5-[2-(4-{[5-({4-[2-(1,4-Dimethyl-1H-1,2,4-triazol-4-ium-5-yl)diazen-1-yl]phenyl}-(ethyl)amino)pentyl](ethyl)amino}phenyl)diazen-1-yl]-1,4-dimethyl-1H-1,2,4-triazol-4-iums
- Salzen des 5-[2-(4-{[3-({4-[2-(1,4-Diethyl-1H-1,2,4-triazol-4-ium-5-yl)diazen-1-yl]phenyl}-amino)propyl]amino}phenyl)diazen-1-yl]-1,4-diethyl-1H-1,2,4-triazol-4-iums
- Salzen des 5-[2-(4-{[4-({4-[2-(1,4-Diethyl-1H-1,2,4-triazol-4-ium-5-yl)diazen-1-yl]phenyl}-amino)butyl]amino}phenyl)diazen-1-yl]-1,4-diethyl-1H-1,2,4-triazol-4-iums
- Salzen des 5-[2-(4-{[5-({4-[2-(1,4-Diethyl-1H-1,2,4-triazol-4-ium-5-yl)diazen-1-yl]phenyl}amino)pentyl]amino}phenyl)diazen-1-yl]-1,4-diethyl-1H-1,2,4-triazol-4-iums
- Salzen des 5-[2-(4-{[3-({4-[2-(1,4-Diethyl-1H-1,2,4-triazol-4-ium-5-yl)diazen-1-yl]phenyl}-(methyl)amino)propyl](methyl)amino}phenyl)diazen-1-yl]-1,4-diethyl-1H-1,2,4-triazol-4-iums
- Salzen des 5-[2-(4-{[4-({4-[2-(1,4-Diethyl-1H-1,2,4-triazol-4-ium-5-yl)diazen-1-yl]phenyl}-(methyl)amino)butyl](methyl)amino}phenyl)diazen-1-yl]-1,4-diethyl-1H-1,2,4-triazol-4-iums
- Salzen des 5-[2-(4-{[5-({4-[2-(1,4-Diethyl-1H-1,2,4-triazol-4-ium-5-yl)diazen-1-yl]phenyl}-(methyl)amino)pentyl](methyl)amino}phenyl)diazen-1-yl]-1,4-diethyl-1H-1,2,4-triazol-4-iums
- Salzen des 5-[2-(4-{[3-({4-[2-(1,4-Diethyl-1H-1,2,4-triazol-4-ium-5-yl)diazen-1-yl]phenyl}-(ethyl)amino)propyl](ethyl)amino}phenyl)diazen-1-yl]-1,4-diethyl-1H-1,2,4-triazol-4-iums
- Salzen des 5-[2-(4-{[4-({4-[2-(1,4-Diethyl-1H-1,2,4-triazol-4-ium-5-yl)diazen-1-yl]phenyl}-(ethyl)amino)butyl](ethyl)amino}phenyl)diazen-1-yl]-1,4-diethyl-1H-1,2,4-triazol-4-iums
- Salzen des 5-[2-(4-{[5-({4-[2-(1,4-Diethyl-1H-1,2,4-triazol-4-ium-5-yl)diazen-1-yl]phenyl}-(ethyl)amino)pentyl](ethyl)amino}phenyl)diazen-1-yl]-1,4-diethyl-1H-1,2,4-triazol-4-iums.

5. Mittel nach einem der Ansprüche 1 bis 4, **dadurch gekennzeichnet, dass** es - bezogen auf das Gesamtgewicht des Mittels - einen oder mehrere direktziehende Farbstoffe (a) der Formel (Ia) in einer Gesamtmenge von 0,01 bis 4,5 Gew.-%, bevorzugt von 0,05 bis 2,8 Gew.-%, weiter bevorzugt von 0,1 bis 2,2 Gew.-% und besonders bevorzugt von 0,2 bis 1,2 Gew.-% enthält.

6. Mittel nach einem der Ansprüche 1 bis 5, **dadurch gekennzeichnet, dass** es (b) mindestens ein anionisches Tensid enthält, das ausgewählt ist aus den
- linearen und verzweigten Fettsäuren mit 8 bis 30 C-Atomen,
- Ethercarbonsäuren der Formel R-O-(CH₂-CH₂O)ₓ-CH₂-COOM, in der
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, die durch Veresterung von C₈-C₂₄-Fettsäuren mit dem Natriumsalz der 2-Hydroxyethan-sulfonsäure (Isethionsäure) zugänglich sind,
- Amidethercarbonsäuren, R¹-CO-NR²-CH₂CH₂-O-(CH₂CH₂O)_{y}CH₂COOM, wobei
R¹ für eine C2-C30-Alkylgruppe steht,
y für eine ganze Zahl von 1 bis 20 steht,
R² für Wasserstoff, einen Methyl-, Ethyl-, Propyl-, Isopropyl-, n-Butyl-, t-Butyl- oder iso-Butylrest steht, und
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

7. Mittel nach einem der Ansprüche 1 bis 6, **dadurch gekennzeichnet, dass** es als anionisches Tensid (b) mindestens eine Ethercarbonsäure der Formel (B1) enthält,
R-O-(CH2-CH2O)x-CH2-COOM (B1),
wobei
R für eine lineare Alkylgruppe mit 8 bis 30 C-Atomen steht,
x für eine ganze Zahl von 0 bis 16, bevorzugt für eine ganze Zahl von 5 bis 11, steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH₄⁺) steht.

8. Mittel nach einem der Ansprüche 1 bis 7, **dadurch gekennzeichnet, dass** es - bezogen auf das Gesamtgewicht des Mittels - ein oder mehrere anionische Tenside (b) in einer Gesamtmenge von 0,05 bis 4,5 Gew.-%, bevorzugt von 0,1 bis 3,1 Gew.-%, weiter bevorzugt von 0,15 bis 2,5 Gew.-% und ganz besonders bevorzugt von 0,2 bis 0,9 Gew.-% enthält.

9. Mittel nach einem der Ansprüche 1 bis 8, **dadurch gekennzeichnet, dass** es als kationisches Tensid (b) eine oder mehrere Verbindungen der Formel (B2) enthält, 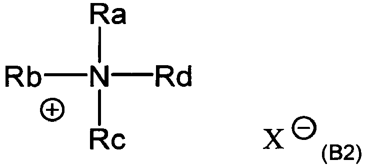 worin
Ra, Rb unabhängig voneinander für eine C₁-C₆-Alkylgruppe, eine C₂-C₆-Alkenylgruppe oder eine C₂-C₆-Hydroxyalkylgruppe stehen,
Rc für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
Rd für eine C₈-C₂₈-Alkylgruppe steht und
X- für ein physiologisch verträgliches Anion steht.

10. Mittel nach einem der Ansprüche 1 bis 9, **dadurch gekennzeichnet, dass** es ein oder mehrere kationische Tenside (b) in einer Gesamtmenge von 0,1 bis 4,8 Gew.-%, bevorzugt von 0,2 bis 2,4 Gew.-%, weiter bevorzugt von 0,3 bis 1,8 Gew.-% - bezogen auf das Gesamtgewicht des Mittels - enthält.

11. Mittel nach einem der Ansprüche 1 bis 10, **dadurch gekennzeichnet, dass** es zusätzlich mindestens einen weiteren kationischen direktziehenden Farbstoff enthält, der von den Farbstoffen der Formel (la) verschieden ist.

12. Verwendung von einem oder mehreren anionischen Tensiden (b) der Formel (B1)
R-O-(CH2-CH2O)x-CH2-COOM (B1),
wobei
R für eine lineare Alkylgruppe mit 8 bis 30 C-Atomen steht,
x für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht,
zur Verbesserung des Farbaufzugsvermögens von direktziehenden Farbstoffen, wie sie in den Ansprüchen 1 bis 4 beschrieben sind, auf keratinischen Fasern.

13. Verwendung von einem oder mehreren katonischen Tensiden (b) der Formel (B2), 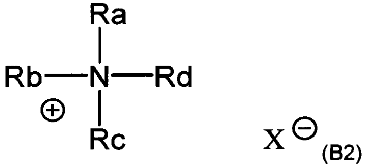 worin
Ra, Rb unabhängig voneinander für eine C₁-C₆-Alkylgruppe, eine C₂-C₆-Alkenylgruppe oder eine C₂-C₆-Hydroxyalkylgruppe stehen,
Rc für eine C₁-C₇-Alkylgruppe, eine C₈-C₂₈-Alkylgruppe, eine C₂-C₆-Alkenylgruppe, oder eine C₂-C₆-Hydroxyalkylgruppe steht,
Rd für eine C₈-C₂₈-Alkylgruppe steht und
X- für ein physiologisch verträgliches Anion steht.
zur Verbesserung des Farbaufzugsvermögens von direktziehenden Farbstoffen, wie sie in den Ansprüchen 1 bis 4 beschrieben sind, auf keratinischen Fasern.

## Claims

1. An agent for dyeing keratin fibers, in particular human hair, containing, in a cosmetic carrier,
(a) at least one substantive dye of formula (Ia), 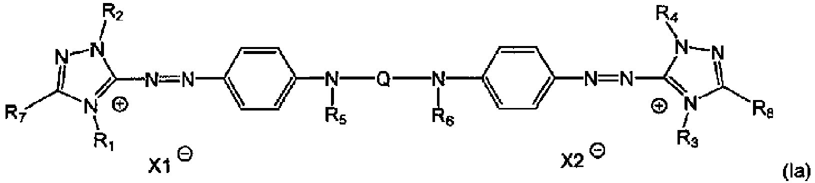 where
R1, R2, R3, R4 represent, independently of one another, a C₁-C₆ alkyl group, a C₂-C₆ alkenyl group or a hydroxy C₂-C₆ alkyl group,
R5, R6 represent, independently of one another, a hydrogen atom, a C₁-C₆ alkyl group, a C₂-C₆ alkenyl group, a hydroxy C₂-C₆ alkyl group or a cyano C₁-C₆ alkyl group,
R7, R8 represent, independently of one another, a hydrogen atom, a C₁-C₆ alkyl group, a halogen atom from the group of chlorine, bromine, fluorine or iodine, or a C₁-C₆ alkoxy group,
X1, X2 represent, independently of one another, a physiologically acceptable anion, preferably from the group of chloride, bromide, iodide, methyl sulfate, methyl sulfonate, p-toluenesulfonate, acetate, hydrogen sulfate, ½ sulfate or ½ tetrachlorozincate,
Q represents a grouping of formula (II), 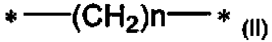
n represents an integer from 3 to 6,
and
(b) at least one anionic surfactant and/or at least one cationic surfactant.

2. The agent according to claim 1, **characterized in that** it contains (a) at least one substantive dye of general formula (la), where
R1, R2, R3, R4 represent, independently of one another, a methyl group or an ethyl group and
R5, R6 represent, independently of one another, a hydrogen atom, a methyl group or an ethyl group.

3. The agent according to one of claims 1 or 2, **characterized in that** it contains (a) at least one substantive dye of general formula (Ia), where
R7, R8 represent, independently of one another, a hydrogen atom, a methyl group, or a methoxy group, but preferably a hydrogen atom.

4. The agent according to one of claims 1 to 3, **characterized in that** it contains (a) at least one substantive dye of general formula (Ia), selected from
- salts of 5-[2-(4-{[3-({4-[2-(1,4-dimethyl-1H-1,2,4-triazol-4-ium-5-yl)diazen-1-yl]phenyl}amino)propyl]amino}phenyl)diazen-1-yl]-1,4-dimethyl-1H-1,2,4-triazol-4-ium
- salts of 5-[2-(4-([4-({4-[2-(1,4-dimethyl-1H-1,2,4-triazol-4-ium-5-yl)diazen-1-yl]phenyl}amino)butyl]amino}phenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]phenyl}amino)pentyl]amino}phenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]phenyl}-(methyl)amino)propyl](methyl)amino}phenyl)diazen-1-yl]-1,4-dimethyl-1H-1,2,4-triazol-4-ium
- salts of 5-[2-(4-{[4-({4-[2-(1,4-dimethyl-1H-1,2,4-triazol-4-ium-5-yl)diazen-1-yl]phenyl}-(methyl)amino)butyl](methyl)amino}phenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]phenyl}-(methyl)amino)pentyl](methyl)amino}phenyl)diazen-1-yl]-1,4-dimethyl-1H-1,2,4-triazol-4-ium
- salts of 5-[2-(4-{[3-({4-[2-(1,4-dimethyl-1H-1,2,4-triazol-4-ium-5-yl)diazen-1-yl]phenyl}-(ethyl)amino)propyl](ethyl)amino}phenyl)diazen-1-yl]-1,4-dimethyl-1H-1,2,4-triazol-4-ium
- salts of 5-(2-(4-{(4-({4-[2-(1,4-dimethyl-1H-1,2,4-triazol-4-ium-5-yl)diazen-1-yl]phenyl}-(ethyl)amino)butyl](ethyl)amino}phenyl)diazen-1-yl]-1,4-dimethyl-1H-1,2,4-triazol-4-ium
- salts of 5-[2-(4-{[5-({4-[2-(1,4-dimethyl-1H-1,2,4-triazol-4-ium-5-yl)diazen-1-yl]phenyl}-(ethyl)amino)pentyl](ethyl)amino}phenyl)diazen-1-yl]-1,4-dimethyl-1H-1,2,4-triazol-4-ium
- salts of 5-[2-(4-{[3-({4-[2-(1,4-diethyl-1H-1,2,4-triazol-4-ium-5-yl)diazen-1-yl]phenyl}-amino)propyl]amino}phenyl)diazen-1-yl]-1,4-diethyl-1H-1,2,4-triazol-4-ium
- salts of 5-[2-(4-{[4-({4-[2-(1,4-diethyl-1H-1,2,4-triazol-4-ium-5-yl)diazen-1-yl]phenyl}-amino)butyl]amino}phenyl)diazen-1-yl]-1,4-diethyl-1H-1,2,4-triazol-4-ium
- salts of 5-[2-(4-{[5-({4-[2-(1,4-diethyl-1H-1,2,4-triazol-4-ium-5-yl)diazen-1-yl]phenyl}amino)pentyl]amino}phenyl)diazen-1-yl]-1,4-diethyl-1H-1,2,4-triazol-4-ium
- salts of 5-[2-(4-{[3-({4-[2-(1,4-diethyl-1H-1,2,4-triazol-4-ium-5-yl)diazen-1-yl]phenyl}-(methyl)amino)propyl](methyl)amino}phenyl)diazen-1-yl]-1,4-diethyl-1H-1,2,4-triazol-4-ium
- salts of 5-[2-(4-{[4-({4-[2-(1,4-diethyl-1H-1,2,4-triazol-4-ium-5-yl)diazen-1-yl]phenyl}-(methyl)amino)butyl](methyl)amino}phenyl)diazen-1-yl]-1,4-diethyl-1H-1,2,4-triazol-4-ium
- salts of 5-[2-(4-{[5-({4-[2-(1,4-diethyl-1H-1,2,4-triazol-4-ium-5-yl)diazen-1-yl]phenyl}-(methyl)amino)pentyl](methyl)amino}phenyl)diazen-1-yl]-1,4-diethyl-1H-1,2,4-triazol-4-ium
- salts of 5-[2-(4-{[3-({4-[2-(1,4-diethyl-1H-1,2,4-triazol-4-ium-5-yl)diazen-1-yl]phenyl}-(ethyl)amino)propyl](ethyl)amino}phenyl)diazen-1-yl]-1,4-diethyl-1H-1,2,4-triazol-4-ium
- salts of 5-[2-(4-{[4-({4-[2-(1,4-diethyl-1H-1,2,4-triazol-4-ium-5-yl)diazen-1-yl]phenyl}-(ethyl)amino)butyl](ethyl)amino}phenyl)diazen-1-yl]-1,4-diethyl-1H-1,2,4-triazol-4-ium
- salts of 5-[2-(4-{[5-({4-[2-(1,4-diethyl-1H-1,2,4-triazol-4-ium-5-yl)diazen-1-yl]phenyl}-(ethyl)amino)pentyl](ethyl)amino}phenyl)diazen-1-yl]-1,4-diethyl-1H-1,2,4-triazol-4-ium.

5. The agent according to one of claims 1 to 4, **characterized in that** it contains, based on the total weight of the agent, one or more substantive dyes (a) of formula (Ia) in a total amount of from 0.01 to 4.5 wt.%, preferably from 0.05 to 2.8 wt.%, more preferably from 0.1 to 2.2 wt.%, and particularly preferably from 0.2 to 1.2 wt.%.

6. The agent according to one of claims 1 to 5, **characterized in that** it contains (b) at least one anionic surfactant, selected from
- linear and branched fatty acids having 8 to 30 C atoms,
- ether carboxylic acids of formula R-O-(CH₂-CH₂O)ₓ-CH₂-COOM, where
R represents a linear alkyl group having 8 to 30 C atoms,
x represents an integer from 0 to 16,
M represents hydrogen or a metal such as an alkali metal, in particular sodium, potassium, lithium, an alkaline-earth metal, particularly ½ magnesium, ½ calcium, ½ zinc, or an ammonium ion (NH4⁺),
- acyl sarcosides having 8 to 24 C atoms in the acyl group,
- acyl taurides having 8 to 24 C atoms in the acyl group,
- acyl isethionates having 8 to 24 C atoms in the acyl group, which can be obtained by esterification of C₈-C₂₄ fatty acids with the sodium salt of 2-hydroxyethanesulfonic acid (isethionic acid),
- amide ether carboxylic acids, R¹-CO-NR²-CH₂CH₂-O-(CH₂CH₂O)_{y}CH₂COOM, where
R¹ represents a C2-C30 alkyl group,
y represents an integer from 1 to 20,
R² represents hydrogen, a methyl, ethyl, propyl, isopropyl, n-butyl, t-butyl or iso-butyl functional group, and
M represents hydrogen or a metal such as an alkali metal, in particular sodium, potassium, lithium, an alkaline-earth metal, in particular ½ magnesium, ½ calcium, ½ zinc, or an ammonium ion (NH4⁺).

7. The agent according to one of claims 1 to 6, **characterized in that** it contains, as an anionic surfactant (b), at least one ether carboxylic acid of formula (B1),
R-O-(CH2-CH2O)x-CH2-COOM (B1),
where
R represents a linear alkyl group having 8 to 30 C atoms,
x represents an integer from 0 to 16, preferably an integer from 5 to 11,
M represents hydrogen or a metal such as an alkali metal, in particular sodium, potassium, lithium, an alkaline-earth metal, in particular ½ magnesium, ½ calcium, ½ zinc, or an ammonium ion (NH4⁺).

8. The agent according to one of claims 1 to 7, **characterized in that** it contains, based on the total weight of the agent, one or more anionic surfactants (b) in a total amount of from 0.05 to 4.5 wt.%, preferably from 0.1 to 3.1 wt.%, more preferably from 0.15 to 2.5 wt.%, and most particularly preferably from 0.2 to 0.9 wt.%.

9. The agent according to one of claims 1 to 8, **characterized in that** it contains, as an cationic surfactant (b), one or more compounds of formula (B2), 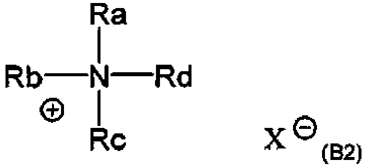 where
Ra, Rb represent, independently of one another, a C₁-C₆ alkyl group, a C₂-C₆ alkenyl group or a C₂-C₆ hydroxyalkyl group,
Rc represents a C₁-C₇ alkyl group, a C₈-C₂₈ alkyl group, a C₂-C₆ alkenyl group, or a C₂-C₈ hydroxyalkyl group,
Rd represents a C₈-C₂₈ alkyl group, and
X- represents a physiologically acceptable anion.

10. The agent according to one of claims 1 to 9, **characterized in that** it contains one or more cationic surfactants (d) in a total amount of from 0.1 to 4.8 wt.%, preferably from 0.2 to 2.4 wt.%, more preferably from 0.3 to 1.8 wt.%, based on the total weight of the agent.

11. The agent according to one of claims 1 to 10, **characterized in that** it additionally contains at least one further cationic substantive dye which is different from the dyes of formula (Ia).

12. The use of one or more anionic surfactants (b) of formula (B1)
R-O-(CH2-CH2O)x-CH2-COOM (B1),
where
R represents a linear alkyl group having 8 to 30 C atoms,
x represents an integer from 0 to 16,
M represents hydrogen or a metal such as an alkali metal, in particular sodium, potassium, lithium, an alkaline-earth metal, particularly ½ magnesium, ½ calcium, ½ zinc, or an ammonium ion (NH4⁺),
for improving the color uptake of substantive dyes, as described in claims 1 to 4, on keratin fibers.

13. The use of one or more cationic surfactants (b) of formula (B2), 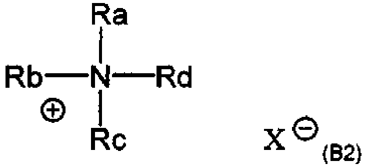 where
Ra, Rb represent, independently of one another, a C₁-C₆ alkyl group, a C₂-C₆ alkenyl group or a C₂-C₆ hydroxyalkyl group,
Rc represents a C₁-C₇ alkyl group, a C₈-C₂₈ alkyl group, a C₂-C₆ alkenyl group, or a C₂-C₆ hydroxyalkyl group,
Rd represents a C₈-C₂₈ alkyl group and
X- represents a physiologically acceptable anion.
for improving the color uptake of substantive dyes, as described in claims 1 to 4, on keratin fibers.

## Revendications

1. Agent de coloration des fibres kératiniques, en particulier de cheveux humains, contenant dans un support cosmétique
(a) au moins un colorant direct de formule (Ia), 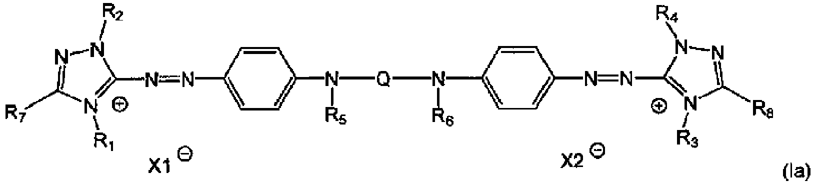 dans laquelle
R1, R2, R3, R4 représentent, indépendamment l'un de l'autre, un groupe alkyle en C₁-C₆, un groupe alcényle en C₂-C₆ ou un groupe hydroxy-alkyle en C₂-C₆,
R5, R6 représentent, indépendamment l'un de l'autre, un atome d'hydrogène, un groupe alkyle en C₁-C₆, un groupe alcényle en C₂-C₆, un groupe hydroxy-alkyle en C₂-C₆ ou un groupe cyano-alkyle en C₁-C₆,
R7, R8 représentent, indépendamment l'un de l'autre, un atome d'hydrogène, un groupe alkyle en C₁-C₆, un atome d'halogène choisi dans le groupe comprenant le chlore, le brome, le fluor ou l'iode, ou un groupe alcoxy en C₁-C₆,
X1, X2 représentent, indépendamment l'un de l'autre, un anion physiologiquement compatible, de manière préférée choisi dans le groupe constitué du chlorure, du bromure, de l'iodure, du sulfate de méthyle, du méthylsulfonate, du p-toluènesulfonate, de l'acétate, de l'hydrogénosulfate, du demi-sulfate ou du demi-tétrachlorozincate,
Q représente un groupe de formule (II), 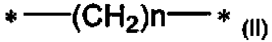
n représente un nombre entier compris entre 3 et 6,
et
(b) au moins un tensioactif anionique et/ou au moins un tensioactif cationique.

2. Agent selon la revendication 1, **caractérisé en ce qu'**il contient (a) au moins un colorant direct de formule générale (Ia), dans laquelle
R1, R2, R3, R4 représentent, indépendamment l'un de l'autre, un groupe méthyle ou un groupe éthyle et
R5, R6 représentent, indépendamment l'un de l'autre, un atome d'hydrogène, un groupe méthyle ou un groupe éthyle.

3. Agent selon l'une quelconque des revendications 1 ou 2, **caractérisé en ce qu'**il contient (a) au moins un colorant direct de formule générale (Ia), dans laquelle
R7, R8 représentent, indépendamment l'un de l'autre, un atome d'hydrogène, un groupe méthyle ou un groupe méthoxy, de manière préférée un atome d'hydrogène.

4. Agent selon l'une quelconque des revendications 1 à 3, **caractérisé en ce qu'**il contient (a) au moins un colorant direct de formule générale (Ia), choisi parmi les
- sels du 5-[2-(4-{[3-({4-[2-(1,4-diméthyl-1H-1,2,4-triazol-4-ium-5-yl)diazène-1-yl]phényl}amino)propyl]amino}phényl)diazène-1-yl]-1,4-diméthyl-1H-1,2,4-triazol-4-ium
- sels du 5-[2-(4-([4-({4-[2-(1,4-diméthyl-1H-1,2,4-triazol-4-ium-5-yl)diazène-1-yl]phényl}amino)butyl]amino}phényl)diazène-1-ylj-1,4-diméthyl-1H-1,2,4-triazol-4-ium
- sels du 5-[2-(4-{[5-({4-[2-(1,4-diméthyl-1H-1,2,4-triazol-4-ium-5-yl)diazène-1-yl]phényl}amino)pentyl]amino}phényl)diazène-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazène-1-yl]phényl}-(méthyl)amino)propyl](méthyl)amino}phényl)diazène-1-yl]-1,4-diméthyl-1H-1,2,4-triazol-4-ium
- sels du 5-[2-(4-{[4-({4-[2-(1,4-diméthyl-1H-1,2,4-triazol-4-ium-5-yl)diazène-1-yl]phényl}-(méthyl)amino)butyl](méthyl)amino}phényl)diazène-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazène-1-yl]phényl}-(méthyl)amino)pentyl](méthyl)amino}phényl)diazène-1-yl]-1,4-diméthyl-1H-1,2,4-triazol-4-ium
- sels du 5-[2-(4-{[3-({4-[2-(1,4-diméthyl-1H-1,2,4-triazol-4-ium-5-yl)diazène-1-yl]phényl}-(éthyl)amino)propyl](éthyl)amino}phényl)diazène-1-yl]-1,4-diméthyl-1H-1,2,4-triazol-4-ium
- sels du 5-(2-(4-{(4-({4-[2-(1,4-diméthyl-1H-1,2,4-triazol-4-ium-5-yl)diazène-1-yl]phényl}-(éthyl)amino)butyl](éthyl)amino}phenyl)diazène-1-yl]-1,4-diméthyl-1H-1,2,4-triazol-4-ium
- sels du 5-[2-(4-{[5-({4-[2-(1,4-diméthyl-1H-1,2,4-triazol-4-ium-5-yl)diazène-1-yl]phényl}-(éthyl)amino)pentyl](éthyl)amino}phényl)diazène-1-yl]-1,4-diméthyl-1H-1,2,4-triazol-4-ium
- sels du 5-[2-(4-{[3-({4-[2-(1,4-diéthyl-1H-1,2,4-triazol-4-ium-5-yl)diazène-1-yl]phényl}-amine)propyl]amino}phényl)diazène-1-yl]-1,4-diéthyl-1H-1,2,4-triazol-4-ium
- sels du 5-[2-(4-{[4-({4-[2-(1,4-diéthyl-1H-1,2,4-triazol-4-ium-5-yl)diazène-1-yl]phényl}-amino)butyl]amino}phényl)diazène-1-yl]-1,4-diéthyl-1H-1,2,4-triazol-4-ium
- sels du 5-[2-(4-{[5-({4-[2-(1,4-diéthyl-1H-1,2,4-triazol-4-ium-5-yl)diazène-1-yl]phényl}amino)pentyl]amino}phényl)diazène-1-yl]-1,4-diéthyl-1H-1,2,4-triazol-4-ium
- sels du 5-[2-(4-{[3-({4-[2-(1,4-diéthyl-1H-1,2,4-triazol-4-ium-5-yl)diazène-1-yl]phényl}-(méthyl)amino)propyl](méthyl)amino}phényl)diazène-1-yl]-1,4-diéthyl-1H-1,2,4-triazol-4-ium
- sels du 5-[2-(4-{[4-({4-[2-(1,4-diéthyl-1H-1,2,4-triazol-4-ium-5-yl)diazène-1-yl]phényl}-(méthyl)amino)butyl](méthyl)amino}phényl)diazène-1-yl]-1,4-diéthyl-1H-1,2,4-triazol-4-ium
- sels du 5-[2-(4-{[5-({4-[2-(1,4-diéthyl-1H-1,2,4-triazol-4-ium-5-yl)diazène-1-yl]phényl}-(méthyl)amino)pentyl](méthyl)amino}phényl)diazène-1-yl]-1,4-diéthyl-1H-1,2,4-triazol-4-ium
- sels du 5-[2-(4-{[3-({4-[2-(1,4-diéthyl-1H-1,2,4-triazol-4-ium-5-yl)diazène-1-yl]phényl}-(éthyl)amino)propyl](éthyl)amino}phényl)diazène-1-yl]-1,4-diéthyl-1H-1,2,4-triazol-4-ium
- sels du 5-[2-(4-{[4-({4-[2-(1,4-diéthyl-1H-1,2,4-triazol-4-ium-5-yl)diazène-1-yl]phényl}-(éthyl)amino)butyl](éthyl)amino}phényl)diazène-1-yl]-1,4-diéthyl-1H-1,2,4-triazol-4-ium
- sels du 5-[2-(4-{[5-({4-[2-(1,4-diéthyl-1H-1,2,4-triazol-4-iunn-5-yl)diazène-1-yl]phényl}-(éthyl)amino)pentyl](éthyl)amino}phényl)diazène-1-yl]-1,4-diéthyl-1H-1,2,4-triazol-4-ium.

5. Agent selon l'une quelconque des revendications 1 à 4, **caractérisé en ce qu'**il contient, par rapport au poids total de l'agent, un ou plusieurs colorants directs (a) de formule (Ia) en une quantité totale située dans la plage allant de 0,01 à 4,5 % en poids, de manière préférée de 0,05 à 2,8 % en poids, de manière davantage préférée de 0,1 à 2,2 % en poids et de manière particulièrement préférée de 0,2 à 1,2 % en poids.

6. Agent selon l'une quelconque des revendications 1 à 5, **caractérisé en ce qu'**il contient (b) au moins un tensioactif anionique, choisi parmi les
- acides gras linéaires et ramifiés ayant 8 à 30 atomes de carbone,
- acides éther carboxyliques de formule R-O-(CH₂-CH₂O)ₓ-CH₂-COOM, dans laquelle
R représente un groupe alkyle linéaire ayant 8 à 30 atomes de carbone,
x représente un nombre entier compris entre 0 et 16,
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺),
- les acylsarcosides ayant 8 à 24 atomes de carbone dans le groupe acyle,
- les acyltaurates ayant 8 à 24 atomes de carbone dans le groupe acyle,
- les acyliséthionates ayant 8 à 24 atomes de carbone dans le groupe acyle, lesquels sont obtenus par estérification des acides gras C₈-C₂₄ avec du sel de sodium de l'acide 2-hydroxyéthane-sulfonique (acide iséthionique),
- les acides amidoéthercarboxyliques de formule R¹-CO-NR²-CH₂CH₂-O-(CH₂CH₂O)_{y}CH₂COOM, dans laquelle
R¹ représente un groupe alkyle C2-C30,
y représente un nombre entier compris entre 1 et 20,
R² représente un atome d'hydrogène, un radical méthyle, éthyle, propyle, isopropyle, n-butyle, t-butyle ou isobutyle, et
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺).

7. Agent selon l'une quelconque des revendications 1 à 6, **caractérisé en ce qu'**il contient, comme tensioactif anionique (b), au moins un acide éther carboxylique de formule (B1),
R-O-(CH2-CH2O)x-CH2-COOM (B1),
dans laquelle
R représente un groupe alkyle linéaire ayant 8 à 30 atomes de carbone,
x représente un entier entre 0 et 16, de manière préférée entre 5 et 11,
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺).

8. Agent selon l'une quelconque des revendications 1 à 7, **caractérisé en ce qu'**il contient, par rapport au poids total de l'agent, un ou plusieurs colorants directs (b) en une quantité totale située dans la plage allant de 0,05 à 4,5 % en poids, de préférence de 0,1 à 3,1 % en poids, de manière davantage préférée de 0,15 à 2,5 % en poids et de manière particulièrement préférée de 0,2 à 0,9 % en poids.

9. Agent selon l'une quelconque des revendications 1 à 8, **caractérisé en ce qu'**il contient comme tensioactif cationique (b) un ou plusieurs composés de formule (B2), 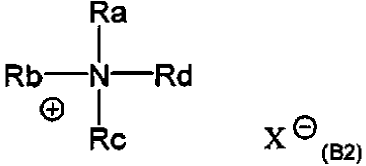 dans laquelle
Ra, Rb représentent, indépendamment l'un de l'autre, un groupe alkyle en C₁-C₆, un groupe alcényle en C₂-C₆ ou un groupe hydroxyalkyle en C₂-C₆,
Rc représente un groupe alkyle en C₁-C₇, un groupe alkyle en C₈-C₂₈, un groupe alcényle en C₂-C₆ ou un groupe hydroxyalkyle en C₂-C₈,
Rd représente un groupe alkyle en C₈-C₂₈ et
X- représente un anion physiologiquement compatible.

10. Agent selon l'une quelconque des revendications 1 à 9, **caractérisé en ce qu'**il contient, un ou plusieurs tensioactifs cationiques (b) en une quantité totale allant de 0,1 à 4,8 % en poids, de manière préférée de 0,2 à 2,4 % en poids, de manière davantage préférée de 0,3 à 1,8 % en poids, rapporté au poids total de l'agent.

11. Agent selon l'une quelconque des revendications 1 à 10, **caractérisé en ce qu'**il contient en outre au moins un colorant direct cationique supplémentaire, qui est différent des colorants de la formule (la).

12. Utilisation d'un ou de plusieurs tensioactifs anioniques (b) de formule (B1)
R-O-(CH2-CH2O)x-CH2-COOM (B1),
dans laquelle
R représente un groupe alkyle linéaire ayant 8 à 30 atomes de carbone,
x représente un nombre entier compris entre 0 et 16,
M représente l'hydrogène ou un métal tel qu'un métal alcalin, en particulier le sodium, le potassium, le lithium, un métal alcalino-terreux, en particulier le demi-magnésium, le demi-calcium, le demi-zinc ou un ion ammonium (NH4⁺),
pour améliorer la capacité de tenue des couleurs des colorants directs, tels que décrits dans les revendications 1 à 4, sur des fibres kératiniques.

13. Utilisation d'un ou de plusieurs tensioactifs cationiques (b) de formule (B2), 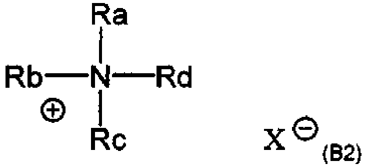 dans laquelle
Ra, Rb représentent, indépendamment l'un de l'autre, un groupe alkyle en C₁-C₆, un groupe alcényle en C₂-C₆ ou un groupe hydroxyalkyle en C₂-C₆,
Rc représente un groupe alkyle en C₁-C₇, un groupe alkyle en C₈-C₂₈, un groupe alcényle en C₂-C₆ ou un groupe hydroxyalkyle en C₂-C₆,
Rd représente un groupe alkyle en C₈-C₂₈ et
X- représente un anion physiologiquement compatible.
pour améliorer la capacité de tenue des couleurs des colorants directs, tels que décrits dans les revendications 1 à 4, sur des fibres kératiniques.
